# EUROPEAN PATENT APPLICATION

(11) **EP 4 552 640 A1**
(43) Date of publication of application: **14.05.2025**
(21) Application number: 23835819.6
(22) Date of filing: 05.07.2023
(51) Int. Cl.: A61K 31/409, A61P 29/00, A61P 39/06, A61P 17/00

(54) **PHARMACEUTICAL COMPOSITION FOR PREVENTING OR TREATING INFLAMMATORY DISEASES COMPRISING PEGYLATED BILIRUBIN**

(30) Priority: 05.07.2022 KR 20220082580; 04.07.2023 KR 20230086395
(71) Applicant: Bilix Co., Ltd., Seoul 06241 (KR)
(72) Inventor: KIM, Myung Lip, Busan 48272 (KR); MA, Sang Ho, Suwon-si, Gyeonggi-do 16438 (KR); SHIN, Duck Hyang, Incheon 22020 (KR); JO, Seung Hyun, Seoul 06327 (KR); SHIN, Yu Na, Seoul 08837 (KR); KIM, Hyun Jin, Seongnam-si, Gyeonggi-do 13623 (KR); ABUZAR, Sharif MD, Suwon-si, Gyeonggi-do 16240 (KR); CHOI, Min Ho, Suwon-si, Gyeonggi-do 16531 (KR)
(74) Representative: Klöckner, Christoph
(86) International application number: PCT/KR2023/009478
(87) International publication number: WO 2024/010353

(57) **Abstract**

The present invention relates to a pharmaceutical composition for preventing or treating inflammatory diseases comprising a compound of chemical formula 1, and to a cosmetic composition comprising the compound. By comprising the compound of chemical formula 1, the present invention can protect cells and reduce inflammation by removing reactive oxygen species and suppressing inflammatory cytokines in a non-toxic manner.

## Description

### [Technical Field]

The present invention relates to a pharmaceutical composition for preventing or treating inflammatory diseases, which includes PEGylated bilirubin.

### [Background Art]

Reactive oxygen or reactive oxygen species (ROS) refer to chemically reactive molecules containing oxygen atoms. ROS are compounds of oxygen generated within living organisms, which include peroxygen ions and hydrogen peroxide and have unpaired electrons. Therefore, it corresponds to oxygen which is highly reactive and has a very strong oxidizing power that can attack biological tissues and damage cells. Such ROS can also be generated during normal metabolic processes, and are reported to play a role in regulating cell signaling and homeostasis.

Damage to normal cells due to an increase in the concentration of ROS are called oxidative stress. Oxidative stress reflects imbalance between the production of ROS and the biological ability to detoxify reaction intermediates or recover damage caused by the ROS. If the imbalance caused by the oxidative stress is severe, cell death may occur. Further, increased ROS may cause direct damage to DNA present in the organism. This causes various inflammatory responses, aging, hyperlipidemia, chronic fatigue, vascular disease, or the like, and may also worsen existing diseases.

### [Summary of Invention]

### [Problems to be Solved by Invention]

An object of the present invention is to provide a pharmaceutical composition for preventing or treating inflammatory diseases, which includes PEGylated bilirubin.

Another object of the present invention is to provide a cosmetic composition which includes PEGylated bilirubin.

### [Means for Solving Problems]

1. A pharmaceutical composition for preventing or treating inflammatory diseases, comprising a compound represented by Formula 1, a solvate thereof, or a pharmaceutically acceptable salt thereof:

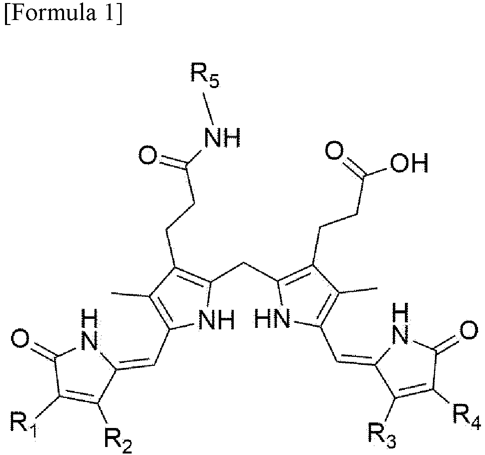

   (in Formula 1 above, R₁ and R₄ are vinyl or methyl groups, and R₂ and R₃ are methyl or vinyl groups, but all of them may not be vinyl or methyl groups; and
   R₅ is polyethylene glycol (PEG) or derivatives thereof).
2. The pharmaceutical composition according to the above 1, wherein the compound is a compound represented by any one of Formulae 2 to 7: 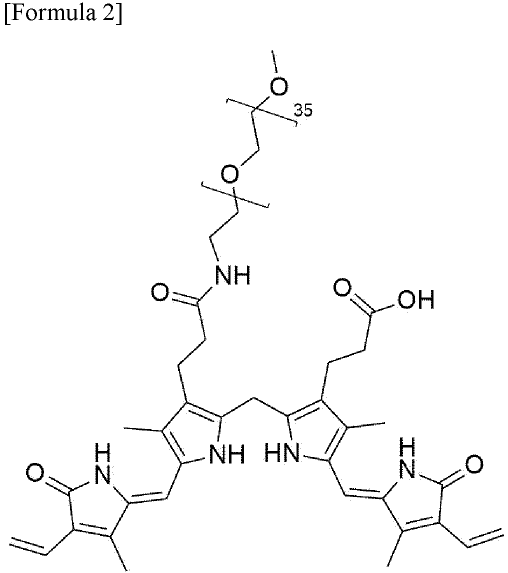 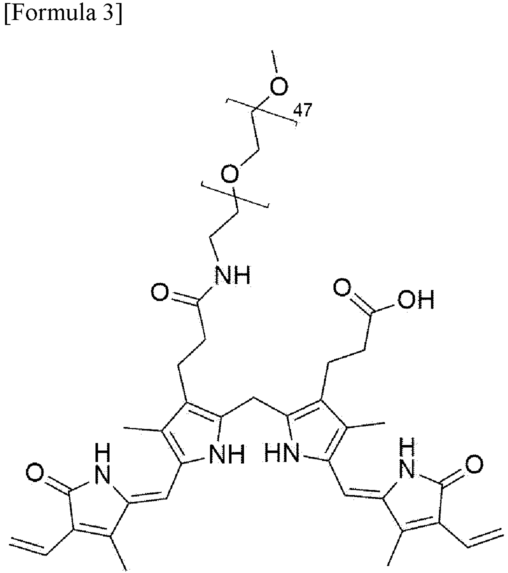 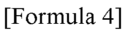 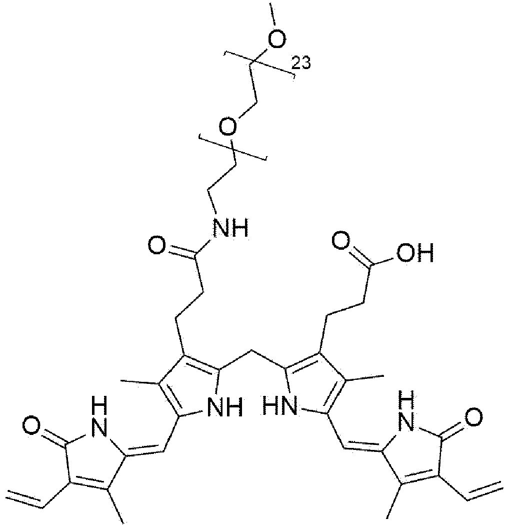 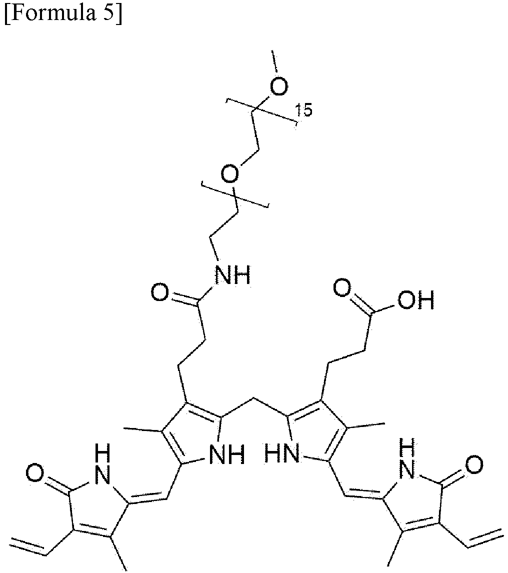 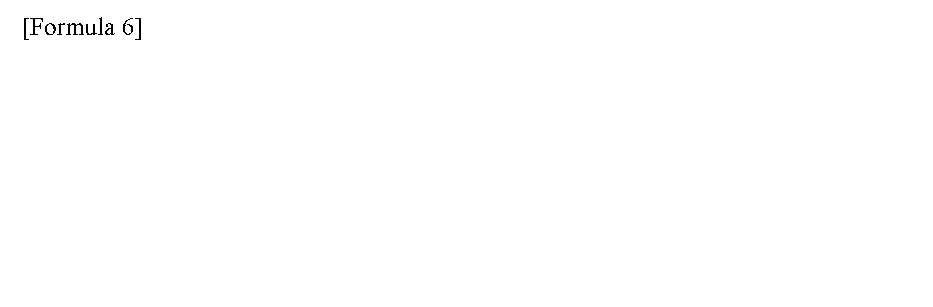 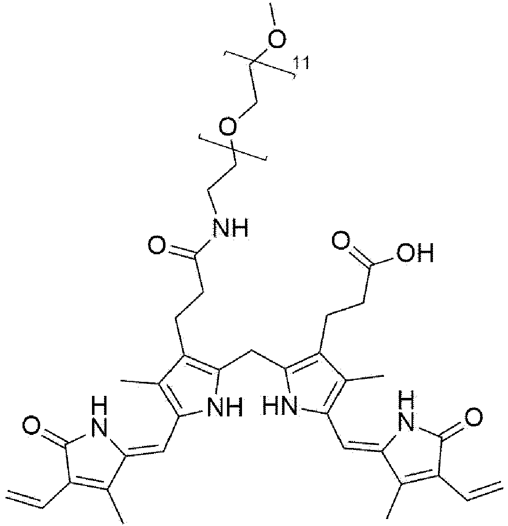 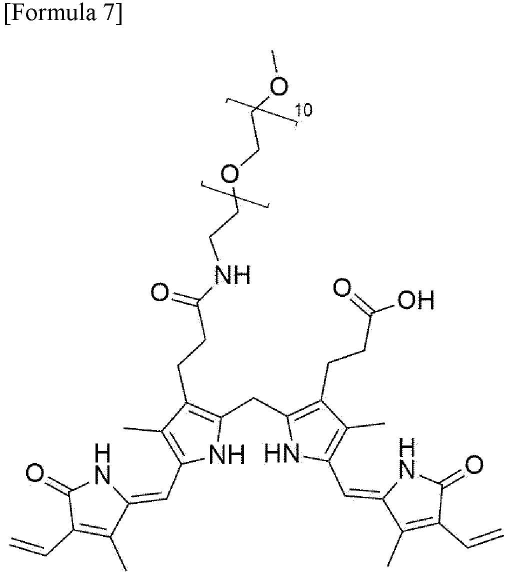
3. The pharmaceutical composition according to the above 1, comprising nanoparticles formed by self-assembly of the compound represented by Formula 1.
4. The pharmaceutical composition according to the above 3, wherein the nanoparticles have a size of 1 to 5000 nm.
5. The pharmaceutical composition according to the above 1, wherein the derivative of polyethylene glycol is selected from the group consisting of methoxy polyethylene glycol (methoxy PEG), succinimide of PEG propionic acid, succinimide of PEG butanoic acid, branched PEG-NHS, PEG succinimidyl succinate, succinimide of carboxymethylated PEG, benzotriazole carbonate of PEG, PEG-glycidyl ether, PEG-oxycarbonylimidazole, PEG nitrophenyl carbonates, PEG-aldehyde, PEG succinimidyl carboxymethyl ester and PEG succinimidyl ester.
6. The pharmaceutical composition according to the above 1, wherein the inflammatory disease is at least one selected from the group consisting of inflammatory skin disease, osteoarthritis, hepatitis, pneumonia, keratitis, gastritis, nephritis, tuberculosis, bronchitis, pleurisy, peritonitis, spondylitis, pancreatitis, inflammatory bowel disease, urethritis, cystitis, inflammatory arteriosclerosis, sepsis, periodontitis, gingivitis and autoinflammatory diseases.
7. The pharmaceutical composition according to the above 6, wherein the inflammatory skin disease is at least one selected from the group consisting of atopic dermatitis, contact dermatitis, psoriasis, seborrheic dermatitis, pruritus, parapsoriasis, urticaria, Lichen planus, sunburn, radiodermatitis, erythema multiforme, erythema nodosum, granuloma annulare, keratosis pilaris, xeroderma, panniculitis, pyoderma gangrenosum, acne, rosacea, lupus erythematosus, pemphigus, diaper dermatitis, pityriasis rosea, alopecia areata, androgenic alopecia, vitiligo and decubitus ulcer.
8. The pharmaceutical composition according to the above 1, wherein the inflammatory disease is caused by increased oxidative stress.
9. A cosmetic composition, comprising a compound represented by Formula 1, a solvate thereof, or a cosmetically acceptable salt thereof: 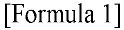 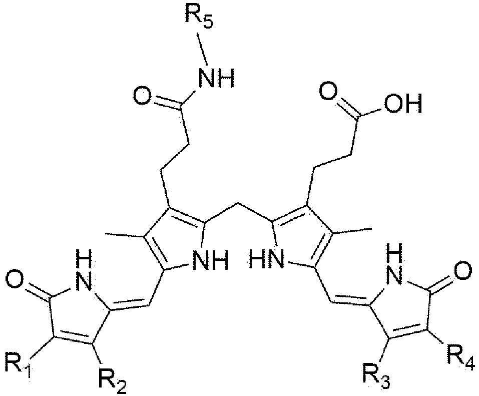
   (in Formula 1 above, R₁ and R₄ are vinyl or methyl groups, and R₂ and R₃ are methyl or vinyl groups, but all of them may not be vinyl or methyl groups; and
   R₅ is polyethylene glycol (PEG) or derivatives thereof).
10. The cosmetic composition according to the above 9, wherein the compound is a compound represented by any one of Formulae 2 to 7 above.
11. The cosmetic composition according to the above 9, comprising nanoparticles formed by self-assembly of the compound represented by Formula 1.
12. The cosmetic composition according to the above 11, wherein the nanoparticles have a size of 1 to 5000 nm.
13. The cosmetic composition according to the above 9, wherein the derivative of polyethylene glycol is selected from the group consisting of methoxy polyethylene glycol (methoxy PEG), succinimide of PEG propionic acid, succinimide of PEG butanoic acid, branched PEG-NHS, PEG succinimidyl succinate, succinimide of carboxymethylated PEG, benzotriazole carbonate of PEG, PEG-glycidyl ether, PEG-oxycarbonylimidazole, PEG nitrophenyl carbonates, PEG-aldehyde, PEG succinimidyl carboxymethyl ester and PEG succinimidyl ester.
14. The cosmetic composition according to the above 9, wherein the cosmetic composition is for anti-inflammatory or anti-oxidation.
15. The cosmetic composition according to the above 9, wherein the cosmetic composition is used to prevent or improve skin inflammation caused by increased oxidative stress.
16. The pharmaceutical composition according to the above 15, wherein skin inflammation caused by the increased oxidative stress is at least one selected from the group consisting of atopic dermatitis, contact dermatitis, psoriasis, seborrheic dermatitis, pruritus, parapsoriasis, urticaria, Lichen planus, sunburn, radiodermatitis, erythema multiforme, erythema nodosum, granuloma annulare, keratosis pilaris, xeroderma, panniculitis, pyoderma gangrenosum, acne, rosacea, lupus erythematosus, pemphigus, diaper dermatitis, pityriasis rosea, alopecia areata, androgenic alopecia, vitiligo and decubitus ulcer.
17. The cosmetic composition according to the above 9, wherein the cosmetic composition is for moisturizing skin, restoring skin barrier function, or preventing skin aging.

### [Advantageous effects]

The composition of the present invention may include PEGylated bilirubin, thereby effectively removing ROS that cause inflammation by selectively targeting inflamed tissues and cells without causing cytotoxicity.

### [Brief Description of Drawings]

FIG. 1 illustrates results of molecular weight analysis (QTOF mass spectrometry) data of the synthesized compounds of Formulae 2 to 7.
FIGS. 2A and 2B illustrate results of confirming whether PEGylated bilirubin nanoparticles are formed using dynamic light scattering (DLS).
FIGS. 3A to 3C illustrate results of confirming whether Dil dye is loaded and whether PEGylated bilirubin nanoparticles are formed using dynamic light scattering (DLS) and absorbance change.
FIGS. 4A and 4B illustrate results of confirming an increase in intracellular influx of Brixelle according to inflammation-inducing conditions (house dust mite (HDM) antigen stimulation) in human keratinocyte cell line (HaCaT).
FIGS. 5A to 5D illustrate results of treating human keratinocyte cell line (HaCaT) with Brixelle under inflammation-inducing conditions (HDM or C48/80 stimulation), thereby confirming that Brixelle can remove reactive oxygen species within skin cells and protect cells.
FIGS. 6A to 6F illustrate results of confirming that Brixelle's dose-dependently suppresses cytokines (TSLP, IL-25, IL-33) produced under inflammation-inducing conditions (HDM or C48/80 stimulation) in human keratinocyte cell line (HaCaT).
FIGS. 7A, 7B (7ba to 7bd) and 7C illustrate results of confirming that the skin barrier function is restored while the inflammatory response is suppressed in a dose-dependent manner by Brixelle treatment using a reconstructed human epidermis model (KeraSkin^{™}) under inflammation-inducing conditions (UVB irradiation).
FIGS. 8A to 8E illustrate results of confirming the dose-dependent therapeutic efficacy of Brixelle for atopic dermatitis by topically treating Brixelle every day for 3 weeks in a house dust mice antigen induced atopic dermatitis mouse model.
FIGS. 9A to 9D illustrate results of confirming the therapeutic efficacy of Brixelle topically treated every day for 3 weeks in a house dust mite antigen induced atopic dermatitis mouse model by comparing with drugs (Elidel, Dexamethasone, Eucrisa) used to treat atopic dermatitis.
FIGS. 10A to 10D illustrate results of confirming the therapeutic efficacy for psoriasis by topically treating Brixelle every day for 7 days in an imiquimod (IMQ) induced psoriasis mouse model.
FIGS. 11A to 11D illustrate results of confirming the therapeutic efficacy for contact dermatitis by topically treating Brixelle every day for 5 days in a DNFB (2,4-dinitrofluorobenzene) induced contact dermatitis mouse model.
FIGS. 12A to 12C, 12D (12da and 12db) and 12E illustrate results of confirming that Brixelle is a substance with a very low risk of skin toxicity by performing skin irritation test, eye irritation test, skin micronucleus test, skin sensitization test, and skin phototoxicity test through an alternative animal test method using a reconstructed human epidermis model (KeraSkin^{™}) and a reconstructed human cornea-like epithelium model (MCTT HCE^{™}).

### [Mode for Carrying out Invention]

Hereinafter, the present invention will be described in detail.

The present invention relates to a pharmaceutical composition for preventing or treating inflammatory diseases, which includes: a compound represented by Formula 1, a solvate thereof, or a pharmaceutically acceptable salt thereof.

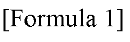

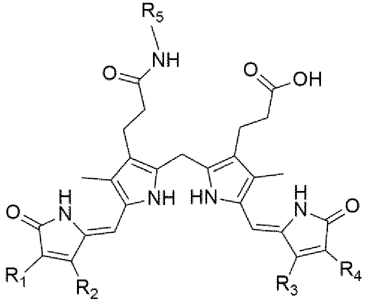

In Formula 1 above, R₁ and R₄ are vinyl or methyl groups, R₂ and R₃ are methyl or vinyl groups, but all of them may not be vinyl groups or methyl groups, and R₅ is polyethylene glycol (PEG) or derivatives thereof.

The polyethylene glycol or derivatives thereof may have a molecular weight of 500 to 10000, 500 to 9000, 500 to 8000, 500 to 7000, 500 to 6000, or 500 to 5000, but it is not limited thereto.

The polyethylene glycol or derivatives thereof may be a straight chain or branched chain.

For example, the derivative of polyethylene glycol may be selected from the group consisting of methoxy polyethylene glycol (methoxy PEG), succinimide of PEG propionic acid, succinimide of PEG butanoic acid, branched PEG-NHS, PEG succinimidyl succinate, succinimide of carboxymethylated PEG, benzotriazole carbonate of PEG, PEG-glycidyl ether, PEG-oxycarbonylimidazole, PEG nitrophenyl carbonates, PEG-aldehyde, PEG succinimidyl carboxymethyl ester and PEG succinimidyl ester.

Specifically, the compound may be a compound represented by any one of Formulae 2 to 7 below.

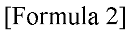

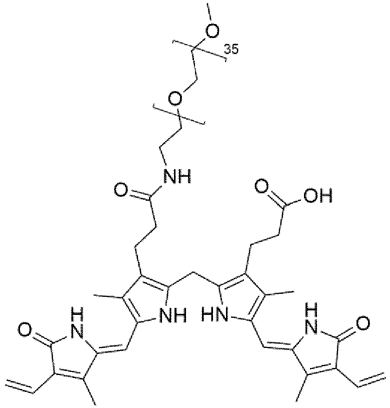

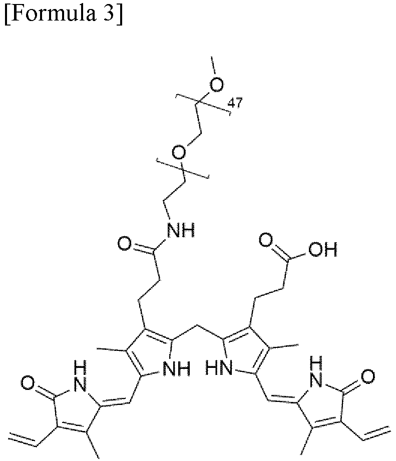

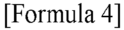

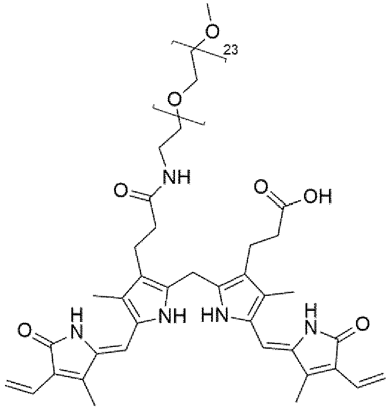

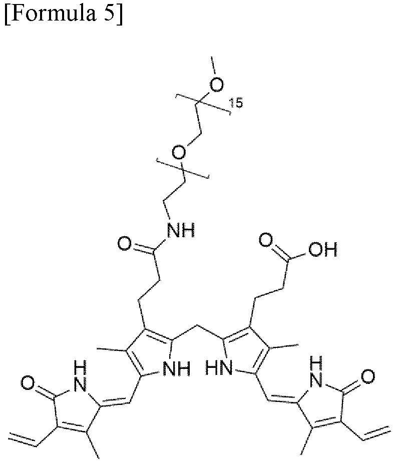

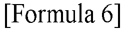

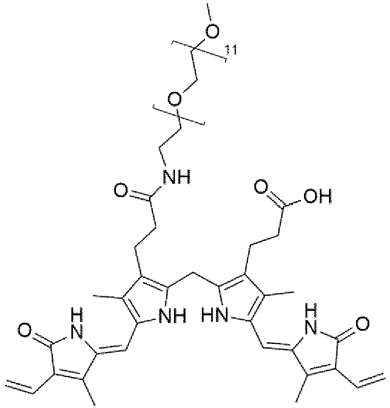

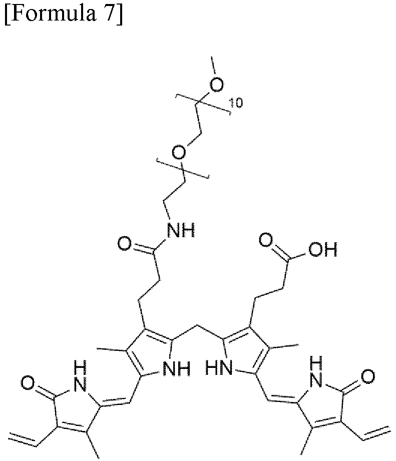

The pharmaceutical composition of the present invention may include nanoparticles formed by self-assembly of the compound represented by Formula 1 above.

The nanoparticles may have a size of 1 to 5000 nm, and more specifically 50 to 1000 nm, 50 to 500 nm, or 50 to 300 nm, but it is not limited thereto.

The inflammatory disease may be at least one selected from the group consisting of inflammatory skin disease, osteoarthritis, hepatitis, pneumonia, keratitis, gastritis, nephritis, tuberculosis, bronchitis, pleurisy, peritonitis, spondylitis, pancreatitis, inflammatory bowel disease, urethritis, cystitis, inflammatory arteriosclerosis, sepsis, periodontitis, gingivitis and autoinflammatory diseases, but it is not limited thereto.

The inflammatory skin disease may be at least one selected from the group consisting of, for example, atopic dermatitis, contact dermatitis, psoriasis, seborrheic dermatitis, pruritus, parapsoriasis, urticaria, Lichen planus, sunburn, radiodermatitis, erythema multiforme, erythema nodosum, granuloma annulare, keratosis pilaris, xeroderma, panniculitis, pyoderma gangrenosum, acne, rosacea, lupus erythematosus, pemphigus, diaper dermatitis, pityriasis rosea, alopecia areata, androgenic alopecia, vitiligo and decubitus ulcer.

Causes of the inflammatory disease may be abnormalities in the immune system, infection, external stimulation or damage, environmental factors, genetic factors, etc., but they are not limited thereto. For example, the inflammatory disease may be caused by increased oxidative stress.

The term "oxidative stress" refers to a phenomenon in which oxidative substances such as reactive oxygen species are overproduced or antioxidant substances are insufficient, thus causing an imbalance and damaging or destroying cells or tissues. Reactive oxygen species (ROS) are chemically reactive molecules containing oxygen atoms, which are a compound of oxygen produced within living organisms as oxygen having a high oxidizing ability that attacks biological tissues and damages cells. ROS are generated during a normal metabolic process of oxygen, but their concentration may be rapidly increased due to environmental stress, etc., thereby causing damage to the cells.

Causes of excessive production of ROS or oxidative stress may include various internal and external causes such as, for example, incorrect eating habits, nutritional imbalance, irregular lifestyle, excessive exercise, smoking, drinking, overwork, immune response, surgery, administration of anticancer drugs, exhaust gases, heavy metals, radiation, UV rays, ultrasound, electromagnetic waves, chemicals (detergents, pesticides, etc.), or the like, but they are not limited thereto.

Further, the inflammatory skin disease may occur due to skin dryness, increased sensitivity, pigmentation, and local inflammation of the skin, which are caused by damage to the barrier function of the epidermis and stratum corneum due to oxidative stress.

The term "prevention" refers to any action that suppresses or delays the onset of the related diseases by administering the composition of the present invention. Those skilled in the art in which the present invention pertains would be able to determine that the related diseases may be prevented by administering the composition of the present invention before or in the early stages of symptom development.

The term "treatment" refers to any action that improves or beneficially changes the symptoms of the related diseases by administering the composition of the present invention, and treatment includes alleviation or improvement of the diseases. Those skilled in the art in which the present invention pertains would be able to know the exact criteria for the diseases and determine the degree of improved, enhanced and the treated diseases.

The term "pharmaceutically acceptable" refers to a feature that does not cause serious stimulation to an individual, cells, tissues, etc. in which a compound or composition is administered, and does not impair biological activity and physical properties of the compound.

The expression "pharmaceutically acceptable salt" refers to a salt prepared using a specific compound according to the present invention, as well as acid or base relatively nontoxic thereto. The pharmaceutically acceptable salt may include, for example, acid addition salts or metal salts.

The acid addition salts may be formed from inorganic acids such as hydrochloric acid, nitric acid, phosphoric acid, sulfuric acid, hydrobromic acid, hydroiodic acid, nitrous or phosphorous acid, aliphatic mono and dicarboxylates, phenyl-substituted alkanoates, hydroxy alkanoates and alkane dioates, and non-toxic organic acids such as aromatic acids, aliphatic and aromatic sulfonic acids. These pharmaceutically non-toxic salts may include sulfate, pyrosulfate, bisulfate, sulfite, bisulfite, nitrate, phosphate, monohydrogen phosphate, dihydrogen phosphate, metaphosphate, pyrophosphate, chloride, bromide, iodide, fluoride, acetate, propionate, decanoate, caprylate, acrylate, formate, isobutyrate, caprate, heptanoate, propiolate, oxalate, malonate, succinate, suberate, sebacate, fumarate, maleate, butine-1,4-dioate, nucleic acid-1,6-dioate, benzoate, chlorobenzoate, methylbenzoate, dinitrobenzoate, hydroxybenzoate, methoxybenzoate, phthalate, terephthalate, benzene sulfonate, toluene sulfonate, chlorobenzene sulfonate, xylene sulfonate, phenylacetate, phenylpropionate, phenylbutyrate, citrate, lactate, β_hydroxybutyrate, glycolate, malate, tartrate, methane sulfonate, propane sulfonate, naphthalene-1-sulfonate, naphthalene-2-sulfonate or mandelate.

The metal salt may be a sodium, potassium or calcium salt. The metal salt may be prepared using a base, for example, alkali-metal or alkaline earth metal salts may be obtained by dissolving the compound in an excess amount of alkali-metal hydroxide or alkaline earth metal hydroxide solution, filtering the non-dissolved compound salt, and evaporating or drying the filtrate.

The route of administration of the pharmaceutical composition of the present invention may be oral administration, or parenteral administration including intraneural, intravenous, intramuscular, intraperitoneal, subcutaneous, rectal, and topical, but it is not limited thereto. The route of administration of the pharmaceutical composition of the present invention may be, for example, parenteral administration, specifically, topical administration such as skin creams, nasal drops, eye drops, ear drops, vaginal tablets, rectal suppositories, etc., and more specifically, may be administration of topical skin application.

The term "administration" refers to introducing a predetermined substance to an individual by an appropriate method, and the term "individual" refers to animals such as rats, mice, livestock, as well as humans who have or may develop the related diseases.

The pharmaceutical composition of the present invention may contain a carrier, excipient and diluent, and may be formulated and used in the form of oral formulations such as powder, granules, tablets, capsules, suspension, emulsion, syrup, aerosol, etc., external applications, suppositories, and sterile injection, but it is not limited thereto.

The carrier, excipient and diluent may include, for example, lactose, dextrose, sucrose, dextrin, maltodextrin, sorbitol, mannitol, xylitol, erythritol, maltitol, starch, acacia rubber, alginate, gelatin, calcium phosphate, calcium silicate, cellulose, methyl cellulose, microcrystalline cellulose, polyvinyl pyrrolidone, water, methylhydroxy benzoate, propylhydroxy benzoate, talc, magnesium stearate and mineral oil, but they are not limited thereto. When formulated, it is prepared using diluents or excipients such as fillers, extenders, binders, wetting agents, disintegrants, and surfactants commonly used in the art, but it is not limited thereto.

Solid preparations for oral administration may include tablets, capsules, pills, granules, etc., and these solid preparations may be prepared by mixing at least one excipient, for example, sucrose, lactose, starch, gelatin, calcium carbonate, etc. Further, in addition to the above excipients, lubricants such as magnesium stearate and talc may be used.

Liquid preparations for oral administration may include aqueous solutions, suspensions, syrups, emulsions, etc., and these liquid preparations may be prepared using various excipients such as wetting agents, sweeteners, flavoring agents, and preservatives, in addition to water and liquid paraffin, etc., which are simple diluents.

Preparations for parenteral administration may include sterilized aqueous solutions, non-aqueous solutions, suspensions, emulsions, lyophilized preparations, and suppositories. The non-aqueous solvent or suspension may include propylene glycol, polyethylene glycol, vegetable oil such as olive oil, or injectable ester such as ethyl oleate. As a base for the suppository, witepsol, macrogol, tween 61, cacao, laurin, glycerogelatin, and the like may be used.

Preparations for parenteral administration may include, for example, sterile injectable preparations. The sterile injectable preparations may be aqueous or oily suspensions. The suspension may be formulated according to techniques well known in the art using suitable dispersing agents, wetting agents (e.g. Tween 80) or suspending agents. The sterile injectable preparation may also be a sterile injectable solution or suspension in a non-toxic parenterally acceptable diluent or solvent. Here, the solvents such as mannitol, water, Ringer's solution, or isotonic sodium chloride solution may be used. Further, sterilized non-volatile oils may typically be used as a solvent or suspending medium, and the non-volatile oils may be used without limitation as long as they are less irritating, such as synthetic mono- or diglycerides, etc.

Preparations for topical administration during parenteral administration may include external applications (coating agents), eye drops, nasal drops, inhalation preparations, vaginal tablets, suppositories, and oral rinses. For example, when formulated for external applications, it may contain fatty substances, organic solvents, solubilizers, thickening agents and gelling agents, emollients, antioxidants, suspending agents, stabilizers, foaming agents, fragrances, surfactants, water, ionic emulsifiers, nonionic emulsifiers, fillers, sequestering agents, chelating agents, preservatives, vitamins, blocking agents, wetting agents, essential oils, dyes, pigments, hydrophilic active agents, lipophilic active agents or lipid vesicles, and other adjuvants commonly used in external skin applications. These external applications may be in the form of ointments, patches, gels, creams, or sprays, but they are not limited thereto.

The pharmaceutical composition of the present invention is administered in a pharmaceutically effective amount. The expression "pharmaceutically effective amount" means an amount sufficient to treat a disease with a reasonable benefit/risk ratio applicable to medical treatment, and an effective dose level may be determined based on the conditions and weight of a patient, a type and severity of patient's disease, drug activity, drug sensitivity, time of administration, route of administration and rate of excretion, factors including drugs used concurrently, and other factors well known in the medical field. The pharmaceutical composition of the present invention may be administered as an individual therapeutic agent or administered in combination with other therapeutic agents for inflammatory diseases or skin diseases in relation to oxidative stress. Further, the composition may be administered sequentially or simultaneously with a conventional therapeutic agent, and may be administered in single or multiple doses. In consideration of all of the above factors, it is important to administer a minimum amount capable of attaining the maximum effect without side effects, such an amount could be easily determined by those skilled in the art.

The effective amount of the pharmaceutical composition of the present invention may vary depending on the patient's age, gender, weight, etc., for example, an amount of 0.01 to 1000 mg/kg/day, more preferably an amount of 0.1 to 500 mg/kg/day once may be administered in once or several times in divided doses. However, since the effective amount may be increased or decreased depending on the route of administration, the number of administration doses, severity of disease, age, sensitivity to the drug, etc., the above dosage does not limit the scope of the present invention in any way.

The present invention relates to a cosmetic composition including a compound represented by Formula 1, a solvate thereof, or a cosmetically acceptable salt thereof.

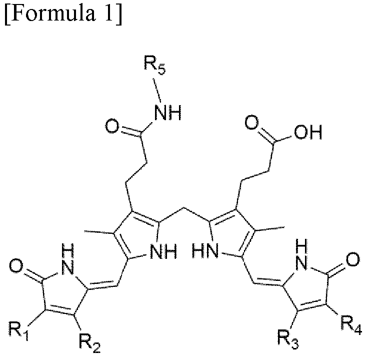

In Formula 1 above, R₁ and R₄ are vinyl or methyl groups, R₂ and R₃ are methyl or vinyl groups, but all of them may not be vinyl or methyl groups, and R₅ is polyethylene glycol (PEG) or derivatives thereof.

The polyethylene glycol or derivatives thereof may be within the above-described range.

The compound may be a compound represented by any one of the above-described Formulae 2 to 7.

The cosmetic composition of the present invention may include nanoparticles formed by self-assembly of the compound represented by Formula 1 above.

Contents regarding the nanoparticles may be within the above-described range.

The cosmetic composition of the present invention may be for anti-inflammatory or anti-oxidation.

Further, the cosmetic composition of the present invention may be used to prevent or improve skin inflammation caused by the increased oxidative stress.

Skin inflammation caused by the increased oxidative stress may occur by skin dryness, increased sensitivity, pigmentation, and local inflammation of the skin, which are caused by damage to the barrier function of the epidermis and stratum corneum due to oxidative stress.

For example, skin inflammation induced by increased oxidative stress may be at least one selected from the group consisting of, for example, atopic dermatitis, contact dermatitis, psoriasis, seborrheic dermatitis, pruritus, parapsoriasis, urticaria, Lichen planus, sunburn, radiodermatitis, erythema multiforme, erythema nodosum, granuloma annulare, keratosis pilaris, xeroderma, panniculitis, pyoderma gangrenosum, acne, rosacea, lupus erythematosus, pemphigus, diaper dermatitis, pityriasis rosea, alopecia areata, androgenic alopecia, vitiligo and decubitus ulcer.

Further, the cosmetic composition of the present invention may be used for moisturizing skin, restoring skin barrier function, or preventing skin aging.

Damage to the skin barrier may occur due to skin aging, ultraviolet rays, hormones, etc. If the skin barrier is damaged, even skin inflammation caused by the increased oxidative stress such as atopic dermatitis and psoriasis may be accompanied.

Skin aging may be induced by UV rays, ROS, dryness, etc. Since the compound of Formula 1 included in the cosmetic composition of the present invention can remove ROS, the cosmetic composition of the present invention may have effects of preventing or recovering skin aging caused by ROS, and skin barrier damage caused by skin aging.

The cosmetic composition of the present invention may be produced in different formulations such as a solution, external ointment, cream, foam, nourishing lotion, softening lotion, pack, softening water, emulsion, makeup base, essence, soap, liquid cleanser, bath agent, sunscreen cream, sun oil, suspension, emulsion, paste, gel, lotion, powder, soap, surfactant-containing cleansing, oil, powder foundation, emulsion foundation, wax foundation, patch or spray, but it is not limited thereto.

The cosmetic composition may include one or more additives selected from: aqueous additives such as purified water, stabilizers, emulsifiers, thickener, moisturizers, liquid crystal film strengtheners, pH regulators, antibacterial agents, water-soluble polymers, coating agents, sequestering agents, amino acids, organic amines, polymer emulsions, skin nutrients, antioxidants, and antioxidant additives, preservatives, flavorings, etc.; and oil-based additives such as fats and oils, waxes, hydrocarbon oils, higher fatty acid oils, higher alcohols, synthetic ester oils, and silicone oils, etc.

The aqueous additive is not limited as long as it is a raw material commonly used in the art, and specific examples may be one or more selected from the group consisting of glycerin, dipropylene glycol, butylene glycol, pentylene glycol, methyl propanediol, sorbitol, diglycerin, erythritol, pentaerythritol, polybutylene glycol-10, polyglycerin-3, polyglycerin-4, polyglycerin-6, polyglycerin-10, polyglycerin-20, polyglycerin-40, sorbeth-5, sorbeth-6, sorbeth-20, sorbeth-30, sorbeth-40, inositol, maltitol, maltose, mannan, mannitol, mannose, lactitol, lactose, dihydroxypropyl PG-glucoside, dithioctanediol, fructose, glucamine, methylglucamine, glucose, 1,2,6-hexanethiol, methylgluceth-10, methylgluceth-20, ozonized glycerin, phytantriol, thioglycerin, threitol, trimethylolpropane, xylitol, EDTA, guar gum, quince seed, carrageenan, galactan, gum arabic, pectin, mannan, starch, xanthan gum, curdlan, methylcellulose, hydroxy ethylcellulose, carboxymethyl cellulose, methyl hydroxypropyl cellulose, chondroitin sulfate, dermatan sulfate, glycogen, heparan sulfate, hyaluronic acid, sodium hyaluronate, gum tragacanth, keratan sulfate, chondroitin, mucoitin sulfate, hydroxyethyl guar gum, carboxymethyl guar gum, dextran, kerato sulfate, locust bean gum, succinoglucan, caronic acid, chitin, chitosan, carboxymethyl chitin, agar, polyvinyl alcohol, polyvinyl pyrrolidone, carboxyvinyl polymer, sodium polyacrylate, polyethylene glycol, bentonite, methylparaben, propylparaben, phenoxyethanol, 1,2-hexanediol, ethylhexyl glycerin or the like.

The oil-based additive is not limited as long as it is a raw material commonly used in the art, and examples thereof may include liquid oils such as olive oil, camellia oil, jojoba oil, triglyceride, glycerin trioctanoate, glycerin triisopalmitate, etc., solid fats such as coconut oil, hydrogenated coconut oil, palm oil, and hydrogenated oil, hydrogenated castor oil, etc., beeswax, candelilla wax, carnauba wax, and lanolin, jojoba wax, etc. Examples of the hydrocarbon oil may include liquid paraffin, squalene, petroleum jelly, and microcrystalline wax. Examples of the higher fatty acid may include waxes such as lauric acid, myristic acid, palmitic acid, stearic acid, behenic acid, etc., and cetyl alcohol, stearyl alcohol, behenyl alcohol, myristyl alcohol, cetostearyl alcohol, etc. The synthetic ester oil may be selected from higher alcohols such as isopropyl myristate, cetyl octanoate, octyl dodecyl myristate, isopropyl palmitate, hexyl laurate, myristyl myristate, cetyl lactate, isocetyl isostearate, neopentyl glycol dicaprate, ethylhexylglycerin, cetylethylhexanoate, ethylhexyl palmitate, cetostearyl alcohol, etc., chain type silicone oils such as dimethylpolysiloxane, methylphenylpolysiloxane, methylhydrogenpolysiloxane, etc., and cyclic silicon oils such as dodecamethyl cyclohexasiloxane., octamethyl cyclotetrasiloxane, decamethylcyclopentasiloxane, etc., but they are not limited thereto.

The cosmetic composition of the present invention may further include a cosmetically acceptable carrier that is commonly mixed with typical skin cosmetics in the art. The cosmetically acceptable carrier may include, for example, oil, water, surfactants, moisturizers, lower alcohols, thickeners, chelating agents, colorants, preservatives, fragrances, etc., but it is not limited thereto.

Depending on the formulation of the cosmetic composition of the present invention, various cosmetically acceptable carriers may be included.

When the formulation of the cosmetic composition is the ointment, paste, cream or gel, animal oil, vegetable oil, wax, paraffin, starch, tragacanth, cellulose derivative, polyethylene glycol, silicone, bentonite, silica, talc, zinc oxide or a mixture thereof may be used as a carrier component.

When the formulation of the cosmetic composition is the powder or spray, lactose, talc, silica, aluminum hydroxide, calcium silicate, polyamide powder or a mixture thereof may be used as the carrier component. In particular, in the case of the spray, the composition may further include propellants such as chloro-fluorohydrocarbon, propane/butane or dimethyl ether.

When the formulation of the cosmetic composition is the solution or emulsion, a solvent, solubilizing agent or emulsifying agent is used as the carrier component. For example, water, ethanol, isopropanol, ethyl carbonate, ethyl acetate, benzyl alcohol, benzyl benzoate, propylene glycol, 1,3-butyl glycol oil, glycerol aliphatic ester, polyethylene glycol or sorbitan fatty acid ester may be used.

When the formulation of the cosmetic composition is the suspension, liquid diluents such as water, ethanol or propylene glycol, suspending agents such as ethoxylated isostearyl alcohol, polyoxyethylene sorbitol esters and polyoxyethylene sorbitan esters, microcrystalline cellulose, aluminum metahydroxide, bentonite, agar, tragacanth, or the like may be used as the carrier component.

When the formulation of the cosmetic composition is the surfactant-containing cleanser, aliphatic alcohol sulfate, aliphatic alcohol ether sulfate, sulfosuccinic acid monoester, isethionate, imidazolinium derivative, methyl taurate, sarcosinate, fatty acid amide ether sulfate, alkylamido betaine, aliphatic alcohol, fatty acid glyceride, fatty acid diethanolamide, vegetable oil, lanolin derivative, ethoxylated glycerol fatty acid ester, or the like may be used as the carrier component.

Ingredients included in the cosmetic composition of the present invention may include, in addition to the above-described compounds and carrier components, components commonly included in the cosmetic compositions, for example, common adjuvants such as antioxidants, stabilizers, solubilizers, vitamins, pigments and fragrances, etc.

The cosmetic composition may be used alone or in multiple applications, or may be used in multiple applications with other cosmetic compositions except for the inventive composition. Further, the cosmetic composition according to the present invention may be used according to any conventional using method, and the number of uses thereof may vary depending on skin conditions or preference of users.

Hereinafter, the present invention will be described in detail with reference to examples.

### Preparative Example

### 1. Preparation of Brixelle

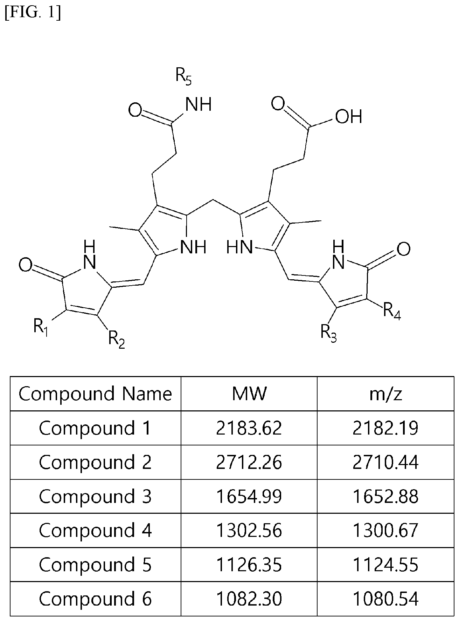

PEGylated bilirubin 3α of Formulae 2 to 7 below (compounds 1 to 6, respectively) were synthesized by varying the molecular weight of PEG, and their molecular weights were analyzed (FIG. 1). Using the synthesized PEGylated bilirubin 3α, Brixelle as its self-assembly product was prepared by the following method.

**[TABLE 1]**

| Formula | Structure | Compound |
|---|---|---|
| 2 | 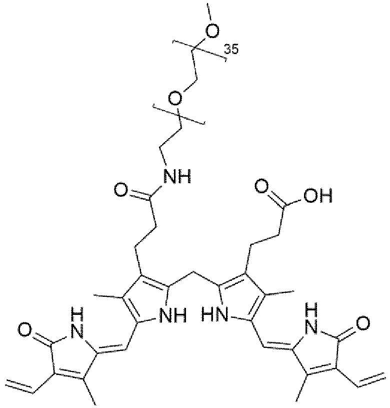 | 1 |
| 3 | 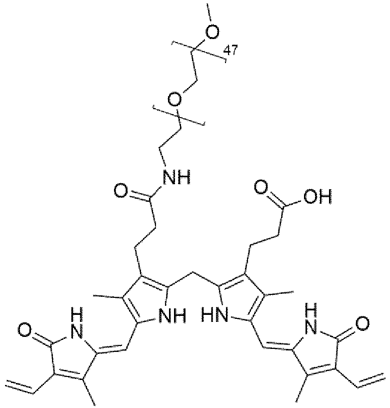 | 2 |
| 4 | 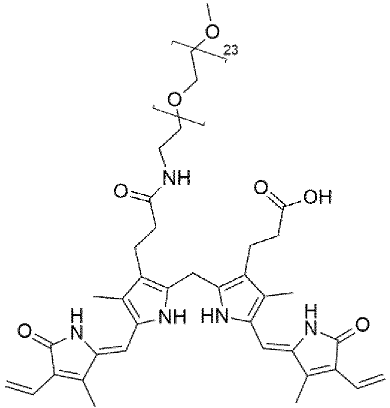 | 3 |
| 5 | 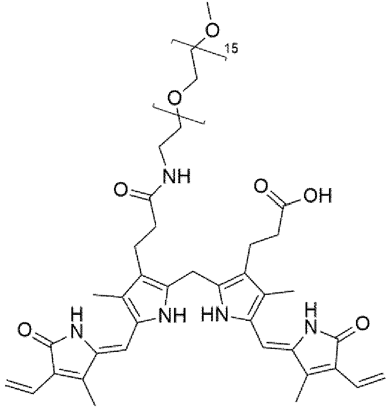 | 4 |
| 6 | 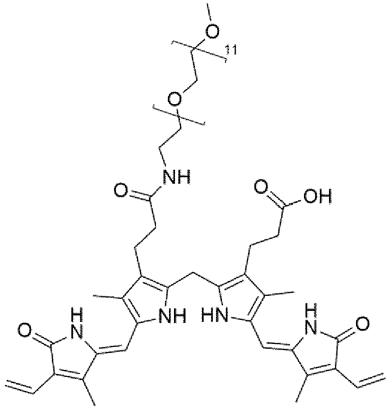 | 5 |
| 7 | 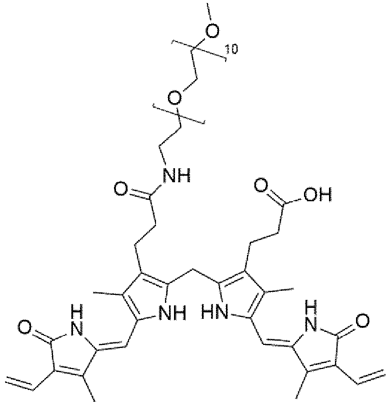 | 6 |

To form Brixelle into nanoparticles of uniform size, it was produced based on flow chemistry.

Specifically, 10 L of citric acid buffer solution was prepared by dissolving sodium citric acid dihydrate and citric acid anhydride in water, and the pH was confirmed to be 4.4. Then, 9.9 L of citric acid buffer solution was prepared in a 30 L reactor. The mixture was stirred at 100 to 1000 rpm while adjusting the temperature to be maintained at 4 to 15 °C.

Next, 1100 mL of acetonitrile was added dropwise to 504.4 g of the PEGylated bilirubin 3α and stirred until completely dissolved. After moving the solution to a 2 L graduated cylinder, acetonitrile was added allowed the total volume to be 1650 mL. The solution was filtered through a 0.20 µm PVDF filter and then transferred to the reactor. The mixture was stirred at 100 to 1000 rpm while adjusting the temperature to be maintained at 4 to 15 °C. The above process was carried out in a yellow light environment.

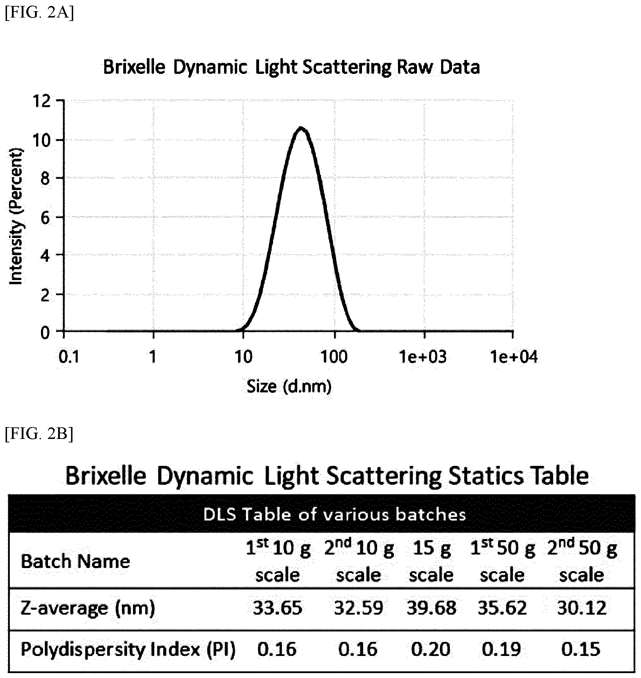

To formulate the product into nanoparticles, 1650 mL of PEGylated bilirubin 3α solution was added to citric acid buffer solution. First, a tube with an inner diameter of 3.2 mm was connected to a pump, and the PEGylated bilirubin 3α solution was flowed into the aqueous solution at a flow rate of 10 to 50 mL/min using this pump and tube. After the dropwise addition of the PEGylated bilirubin 3α solution was completed, the mixture was stirred at 100 to 1000 rpm and 4 to 15 °C for an additional 15 minutes. The above process was also carried out in the yellow light environment. After completion of stirring, the reaction solution was filtered through a 0.20 µm PVDF filter, then transferred to a steel tray, followed by performing lyophilization to obtain Brixelle lyophilized powder. The produced Brixelle powder was re-dissolved in some distilled water, and the formation of nanoparticles was determined using the dynamic light scattering (DLS) (FIGS. 2A and 2B).

### 2. Preparation of tetramethylindocarbocyanine perchlorate(Dil)@Brixelle

To load Dil dye into Brixelle, it was prepared in the following manner.

15 mg of Dil was added to 135 mg of the PEGylated bilirubin 3α, completely dissolved in 6.16 mL of chloroform, filtered through a 0.20 µm PTFE filter, followed by obtaining a thin film using a vacuum rotary evaporator. The thin film was dried under vacuum conditions for 24 hours.

The dried reactant was dissolved in 14.6 mL of distilled water and treated with ultrasonic waves at 20 to 30 °C for 5 to 15 minutes using an ultrasonic water bath to obtain a dispersion. The aqueous solution in which Dil@Brixelle was dispersed was purified by filtering with a 0.45 µm PVDF filter and a 0.20 µm PVDF filter, and then the filtrate was lyophilized to obtain Dil@Brixelle lyophilized powder.

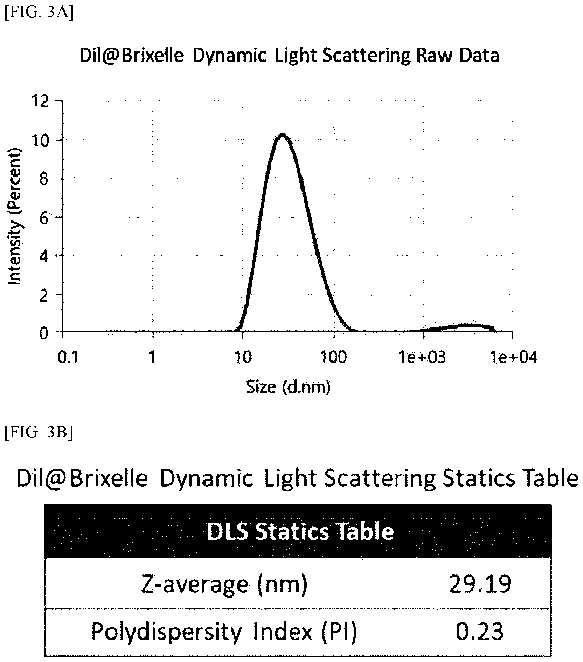

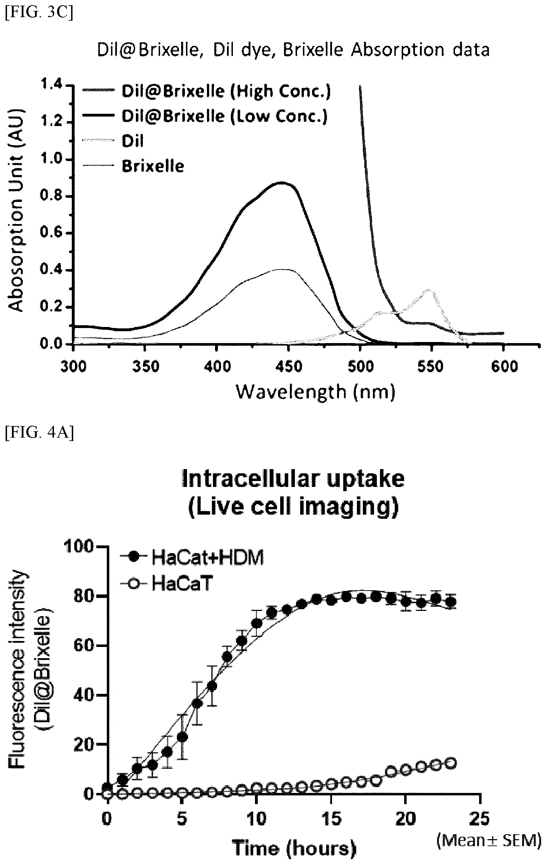

The produced Dil@Brixelle powder was re-dissolved in some distilled water to determine whether Dil@Brixelle nanoparticles were formed using dynamic light scattering (DLS) (FIGS. 3A and 3B). The maximum absorbance of Brixelle and the maximum absorbance of Dil were compared using a multi-mode plate reader to determine whether Dil dye was loaded (FIG. 3C; the graph formed after the wavelength of 500 nm is that of Dil@Brixelle (High Conc.)).

### EXAMPLE

The following experiments were conducted using Brixelle and Dil@Brixelle produced in Preparative Examples 1 and 2 above.

### 1. Results of confirming Brixelle's uptake into skin cells under inflammation-inducing conditions

### (1) Experimental method

To confirm the uptake pattern of Brixelle into skin cells according to inflammation-inducing conditions (house dust mite (HDM) antigen stimulation), a test was conducted using human keratinocytes (HaCaT). HDM antigen is known to induce increased production of reactive oxygen species (ROS) in immune cells and inflammatory cells related to the Th2 immune response in atopic dermatitis and asthma, and is used as a major antigenic stimulation source in an atopic dermatitis-inducing cell model. To establish an inflammation-induced ('inflammatory') cell model, HaCaT cells were treated with 500 µg/mL of HDM antigen for 24 hours. The prepared inflammatory cells were dispensed into a 24-well culture plate and treated with Brixelle loaded with the fluorescent dye tetramethylindocarbocyanine perchlorate (Dil@Brixelle). Thereafter, the intracellular fluorescence expression pattern was observed by a unit of one hour for 24 hours using MuviCyte, a real-time fluorescence microscope equipment.

### (2) Results of experiment

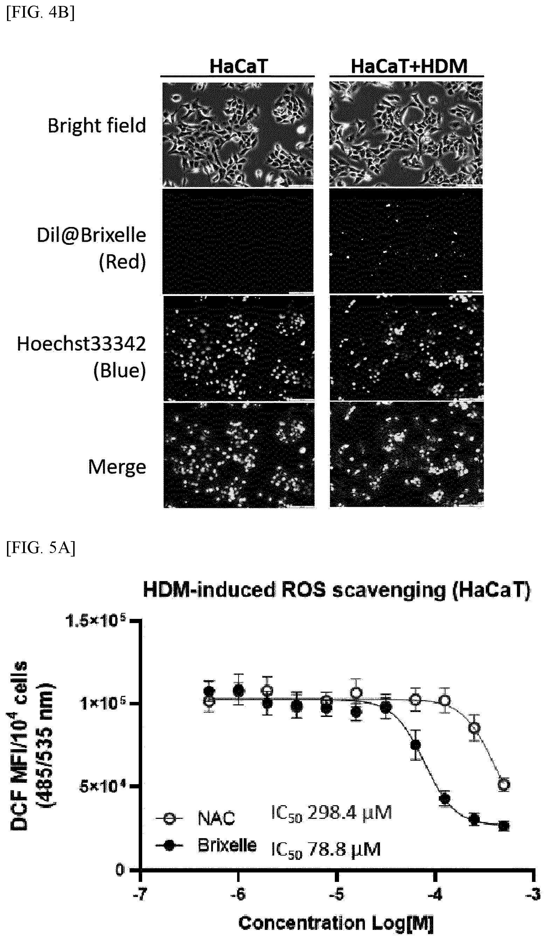

As a result of comparing amounts of Brixelle uptake into HaCaT cells according to inflammation-inducing conditions, a clear increase in Brixelle uptake into inflammatory cells stimulated with HDM antigen was observed compared to the normal cells without HDM antigen stimulation. Specifically, the uptake of Brixelle into the inflammatory cells was increased rapidly from about 4 hours after Brixelle treatment, and was confirmed to be about 6.2 times higher than the amount of Brixelle uptake into the normal cells after 24 hours (FIGS. 4A and 4B).

### 2. Results of confirming Brixelle's reactive oxygen species scavenging ability and cell protection ability within skin cells

### (1) Experimental method

Compound 48/80 (C48/80) is known to mediate inflammatory responses and cell differentiation as well as its role as a mast cell degranulator. In particular, C48/80 has been reported to be involved in skin inflammation through ROS production in keratinocytes and degranulation of mast cells. Therefore, in this test, HDM and C48/80 were used as antigenic and non-antigenic stimulation sources, respectively, in an inflammatory cell model. First, in order to evaluate the ROS scavenging ability of Brixelle in skin cells, HaCaT cells were dispensed into a 96-well black plate, and then treated with HDM and C48/80 at concentrations of 500 µg/mL and 20 µg/mL, respectively, to induce inflammatory stimulation. At the same time, the test substance, Brixelle, and the comparative substance, N-acetylcysteine (NAC), were applied to the cells at different concentrations. After 24 hours, the culture medium was removed and the cells were washed three times with sterilized phosphate-buffered saline (PBS). Again, intracellular ROS was stained by treating the cells with 20 µM of 2',7'-dichlorofluorescin diacetate (DCF-DA) for 45 minutes, and then the fluorescence intensity of the reaction solution was measured in the wavelength range of λex=485/λem=535 nm.

Next, in order to evaluate Brixelle's skin cell protection ability against inflammatory stimulation sources, the cells were treated with antigenic and non-antigenic stimulation sources at a concentration that reduces HaCaT cell viability by 90%, respectively (90% cytotoxic concentration, CC₉₀) (HDM = 2.5 mg/mL, C48/80 = 35 µg/mL). Simultaneously with the treatment of inflammatory stimulation sources, the test substance, Brixelle, and the comparative substance, NAC, were applied to the cells at different concentrations. After 24 hours, the culture medium was removed, washed three times with sterilized PBS, and then replaced with new culture medium. Afterwards, 10 µL of Quanti-Max WST-8 solution corresponding to 1/10 of the culture medium volume was dispensed into each well, and after reaction for 4 hours, cell viability was evaluated by measuring the absorbance of the reaction solution in the wavelength range of 450 nm.

### (2) Results of experiment

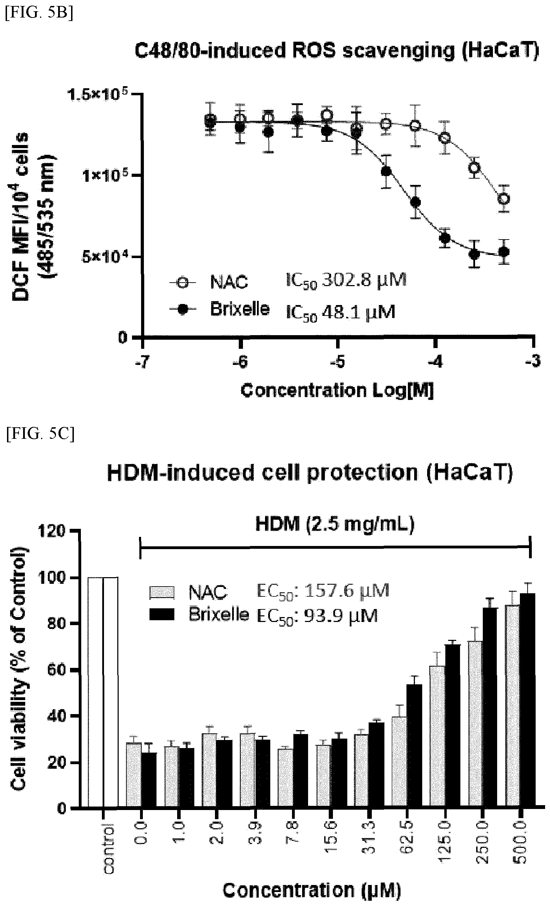

The Brixelle's dose-dependent ROS scavenging effect could be confirmed in inflammatory cell lines induced by the antigenic or non-antigenic stimulation sources. In HDM-induced inflammatory cell lines, the Brixelle's intracellular ROS 50% scavenging ability (50% effective concentration, EC₅₀) was confirmed to be 78.8 µM, which showed about 3.7 times higher than that of NAC, a comparative substance (FIG. 5A). In the C48/80-induced inflammatory cell line, the EC₅₀ value of Brixelle's intracellular ROS scavenging ability was 48.1 µM, which showed about 6.2 times higher than that of NAC (FIG. 5B).

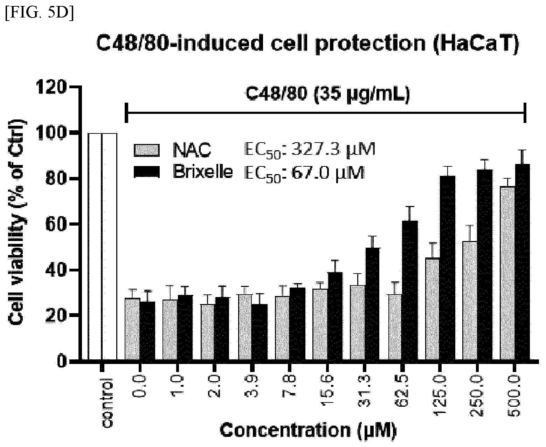

Brixelle showed dose-dependent cell protection effects against both antigenic and non-antigenic stimulation sources in inflammatory cell lines. The EC₅₀ value of Brixelle's cell protection ability in HDM-induced inflammatory cell lines was 93.9 µM, which showed about 1.6 times higher than that of NAC (FIG. 5C). The EC₅₀ value of Brixelle's cell protection ability even in the C48/80-induced inflammatory cell line was 67.0 µM, which showed about 4.8 times higher than that of NAC (FIG. 5D).

Through this, the Brixelle's intracellular ROS scavenging effect was confirmed in an inflammation-induced cell model, and the cell protection effect based on its strong antioxidant effect was demonstrated.

### 3. Results of confirming Brixelle's ability to suppress inflammatory cytokines in skin cells

### (1) Experimental method

Thymic stromal-derived lymphopoietin (TSLP), interleukin (IL)-25 and IL-33 produced from keratinocytes are considered important mediators inducing Th2 immune responses in atopic dermatitis. The effect of Brixelle on the expression of representative inflammatory cytokines secreted by inflammatory cell models induced by the antigenic and non-antigenic stimulation sources was confirmed. First, HaCaT cells were dispensed into a 96-well culture plate, and then treated with HDM and C48/80 at concentrations of 500 µg/mL and 20 µg/mL, respectively. At the same time, the test substance, Brixelle, and the comparative substance, NAC, were applied to the cells at concentrations of 5, 50 and 500 µM concentration. After 24 hours of reaction, the supernatant of the cells was taken and an enzyme-linked immunosorbent assay (ELISA) was performed for each cytokine, and the absorbance of the reaction solution was measured in the wavelength range of 450 nm to quantify an amount of the cytokine secreted by the inflammatory cell line. The results of the graph were expressed as mean ± standard error of the mean (SEM), and statistical analysis was performed using the GraphPad program. The statistical analysis was performed using one-way ANOVA to confirm statistical significance with the negative control group through Dunnett's multiple comparison method (*p<0.05, **p<0.01, ***p<0.001, ****p< 0.0001).

### (2) Results of experiment

The amount of three types of cytokines secreted in the inflammatory cell model induced by HDM and C48/80 stimulation sources showed a statistically significant increase compared to the normal cells (p=0.0006, p<0.0001). Through this, it was confirmed that the inflammatory cell model induced by the stimulation source was suitable for confirming Brixelle's ability to regulate inflammatory cytokines.

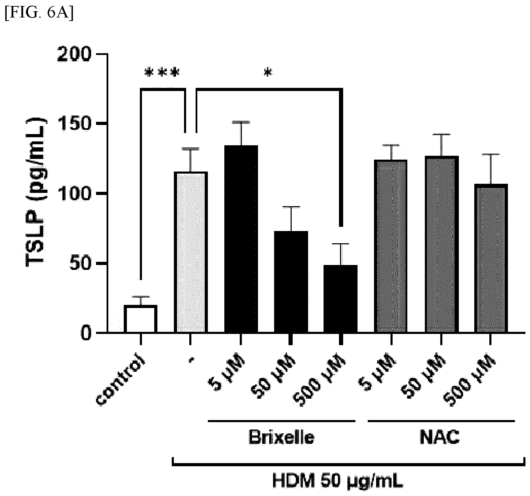

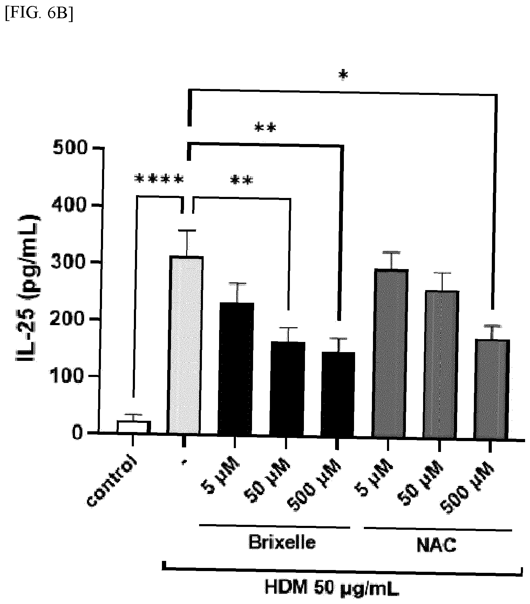

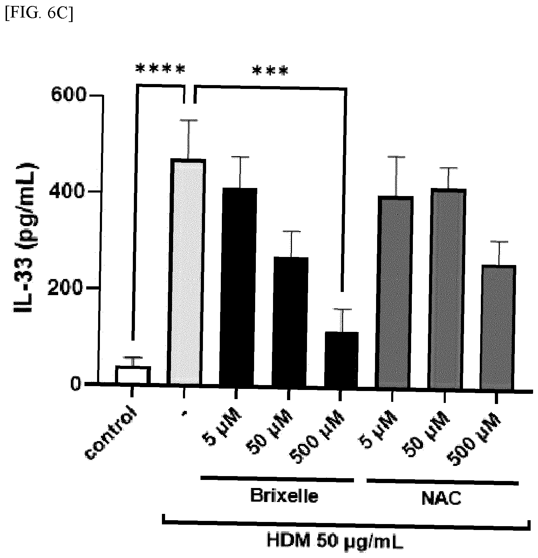

In HDM-induced inflammatory cell lines, the expression levels of all three types of cytokines showed a concentration-dependent decrease trend with respect to Brixelle. In the case of TSLP, there was a 26.4% reduction effect in the Brixelle 50 µM treatment group and a 50.1% reduction effect in the Brixelle 500 µM treatment group compared to the negative control group, while the 500 µM treatment group showed a statistically significant difference from the negative control group (FIG. 6A; p= 0.0224). In the case of IL-25, there was a 47.1% reduction effect in the Brixelle 50 µM treatment group and a 52.5% reduction effect in the Brixelle 500 µM treatment group compared to the negative control group. Both concentration treatment groups showed a statistically significant difference from the negative control group (FIG. 6B; p=0.0054, p=0.0018). IL-33 showed a clear decrease of about 75.1% in the Brixelle 500 µM treatment group compared to the negative control group, and this was confirmed to be a statistically significant difference (FIG. 6C; p=0.001).

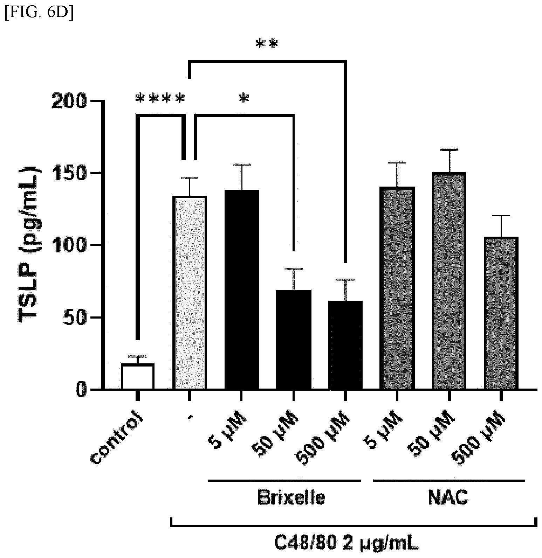

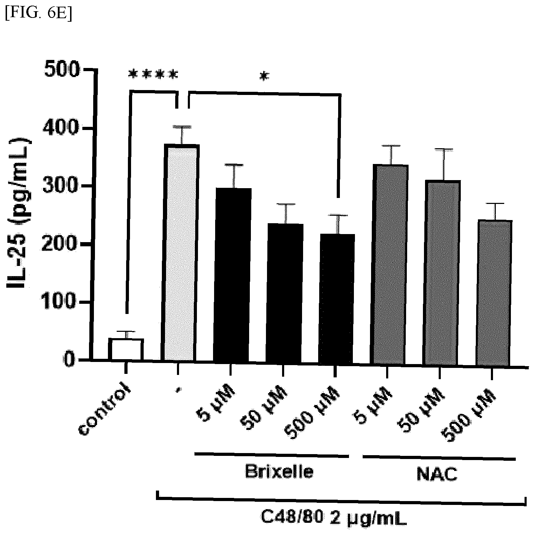

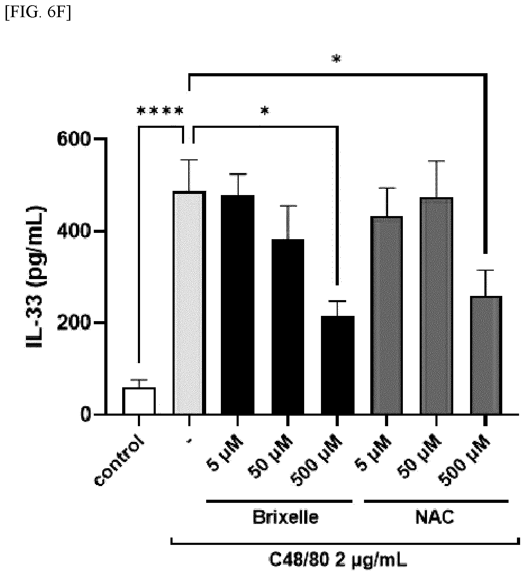

Even in the C48/80-induced inflammatory cell line, the expression levels of all three types of cytokines showed a concentration-dependent reduction trend with respect to Brixelle, and a superior reduction effect was observed compared to NAC at the same concentration. In the case of TSLP, there was a 31.3% reduction effect in the Brixelle 50 µM treatment group and a 38.3% reduction effect in the Brixelle 500 µM treatment group compared to the negative control group. Both concentration treatment groups showed a statistically significant difference from the negative control group (FIG. 6D; p= 0.0143, p=0.0058). In the case of IL-25, there was a 35.2% reduction effect in the Brixelle 50 µM treatment group and a 40% reduction effect in the Brixelle 500 µM treatment group compared to the negative control group, and in the Brixelle 500 µM treatment group, there was a statistically significant difference from the negative control group (FIG. 6E; p=0.0231). In the case of IL-33, there was a 21.1% reduction effect in the Brixelle 50 µM treatment group and a 55.9% reduction effect in the Brixelle 500 µM treatment group compared to the negative control group, and in the Brixelle 500 µM treatment group, there was a statistically significant difference from the negative control group (FIG. 6F; p=0.0120).

Through these results, it was confirmed that Brixelle, which exhibits strong antioxidant effects, has a mechanism to superiorly suppress inflammatory responses in the inflammatory cell lines compared to NAC, a previously known antioxidant.

### 4. Results of confirming Brixelle's inflammation-inhibitory ability in a reconstructed human epidermis model

### (1) Experimental method

It was intended to confirm the Brixelle's inflammation-inhibitory ability in a reconstructed human epidermis model (KeraSkin^{™}) as a human skin tissue model. After adding a reconstructed human epidermis model culture medium to a 6-well culture plate, the prepared artificial skin model (0.6 cm²) was placed and stabilized in a cell incubator at 37 °C and 5% CO₂ for 22±2 hours. To induce an inflammatory response in the reconstructed human epidermis model, a culture plate was inserted into an ultraviolet (UVB) irradiator, and then irradiated with UV rays at 300 mJ/cm². After inducing inflammation, Brixelle 80 and 400 µM were slowly applied to an upper center of the artificial skin model using a pipette and evenly spread over the entire surface. After treating with the test substances in a cell incubator for 8 hours, the test substances were removed using Dulbecco's PBS (DPBS). Thereafter, follow-up culture was performed in a cell incubator for 40 hours, then transepithelial electrical resistance (TEER) and expression of inflammatory cytokines in the culture medium were measured, and tissue staining was conducted after the test was completed, thus to determine effects of inhibiting Brixelle's skin barrier damage.

To evaluate the function and strength of the skin barrier, TEER was measured using an electrical resistance system (ERS-2, Millipore). Further, to confirm changes in inflammatory response, tumor necrosis factor-α (TNF-α), IL-6, IL-1α and TSLP were measured using ELISA. Further, in order to confirm histological changes in the artificial skin model, hematoxylin & eosin (H&E) staining and filaggrin immunohistochemical staining for evaluating skin barrier function were conducted. Results of TEER and inflammatory cytokines were expressed as mean ± standard deviation (SD), and statistical analysis was performed using the GraphPad program. The statistical analysis was performed using one-way ANOVA, and statistical significance between test groups was confirmed through Tukey's multiple comparison method (*p<0.05, **p<0.01, ***p<0.001, ****p<0.0001).

### (2) Results of experiment

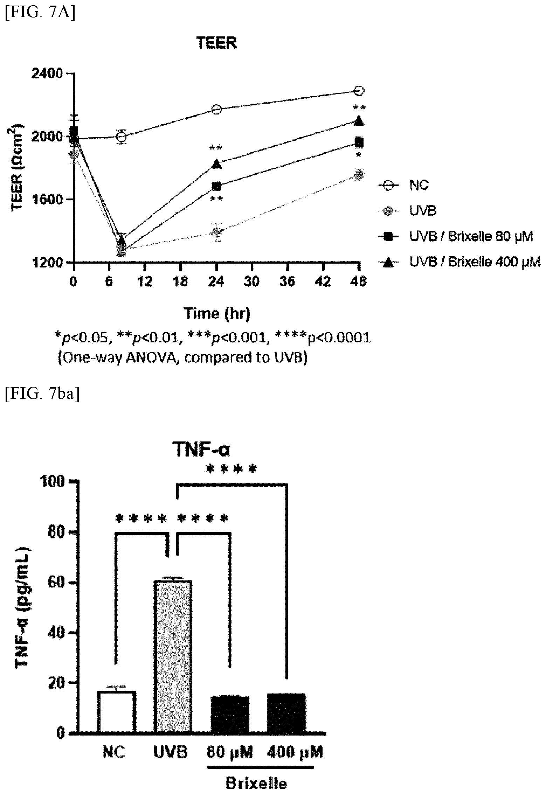

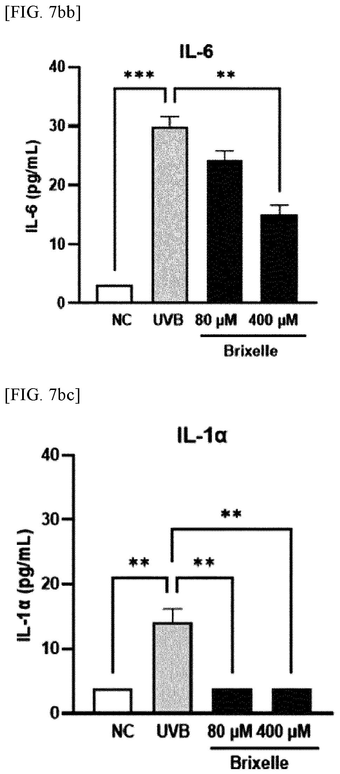

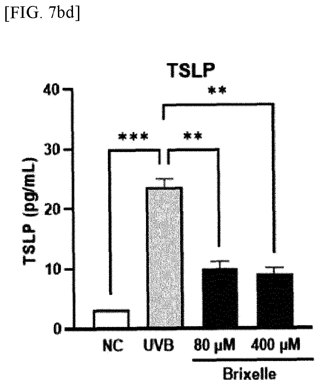

The epithelial cell layer has developed tight junctions, a type of bond between cells, thus to form a membrane-like physicochemical barrier. It is known that, when an inflammatory response occurs in the skin, the skin barrier function is impaired, and TEER is an index to measure the strength of the tight junctions, and it is decreased as the barrier function is impaired. In this experiment, an inflammatory response was induced in the artificial skin model through UV irradiation, and then changes in TEER were analyzed to determine the Brixelle's skin barrier function protection effect. The TEER of the artificial skin model would be decreased compared to the negative control group after UV irradiation, but in the case of the Brixelle treatment group, TEER was significantly recovered in a concentration-dependent manner in the results measured at 24 and 48 hours (FIG. 7A).

It is known that, in various inflammatory diseases occurring in the skin, pro-inflammatory cytokines are expressed at a higher level than in normal skin tissues. In this experiment, among them, TNF-α, IL-6 and IL-1α were analyzed. Further, TSLP, which was found to play an important role in regulating inflammatory responses in association with allergies and skin immune-mediated diseases, was also measured (FIG. 7B (7ba to 7bd)).

As a result of measuring the above four cytokines through ELISA, TNF-α showed a statistically significant increase in the inflammation-induced group, i.e., inflammatory group, compared to the negative control group, but when treated with Brixelle 80 and 400 µM, it was shown that a significant decrease in expression is concentration-dependent (Negative control vs UVB, 16.8±2.6 pg/mL vs 61.1±1.2 pg/mL, p<0.0001; UVB vs Brixelle 80 µM, 61.1±1.2 pg/mL vs 14.7±0.3 pg/mL, p<0.0001; UVB vs Brixelle 400 µM, 61.1±1.2 vs 15.6±0.0, p<0.0001).

As a result of confirming the expression level of IL-6, the p value was 0.0004 and 0.0034, respectively, in the comparison between the negative control group and the inflammation-induced group, and the comparison between the inflammation-induced group and the Brixelle 400 µM treatment group, respectively, which showed a significant decrease in expression level was confirmed compared to the inflammation-induced group when treated with Brixelle (Negative control vs UVB, 3.2±0.0 vs 30.0±2.3, p=0.0004; UVB vs Brixelle 400 µM, 30.0±2.3 vs 15.0±2.3, p=0.0034).

As a result of comparing the expression levels of IL-1α in the culture medium between the IL-1α negative control group and the inflammation-induced group, and between the inflammation-induced group and the Brixelle treatment group 80 and 400 µM, respectively, all the p values between the compared groups were confirmed to be 0.0058 (p<0.01) (Negative control vs UVB, 3.9±0.0 vs 14.1±3.0, p=0.0058; UVB vs Brixelle 80 µM, 14.1±3.0 vs 3.9±0.0, p=0.0058, UVB vs Brixelle 400 µM 14.1±3.0 vs 3.9±0.0, p=0.0058).

Further, the expression level of TSLP, which regulates the inflammatory response, was confirmed to be 3.3±0.0, 23.7±1.9, 10.1±1.6, and 9.1±1.4 pg/mL in the negative control group, inflammation-induced group, and Brixelle 80 µM and 400 µM treated groups, respectively. As a result of comparing the expression levels between the inflammation-induced group and each group, p values satisfied p<0.001, p<0.01 and p<0.01 (Negative control vs UVB, p=0.0004; UVB vs Brixelle 80 µM, p=0.0020; UVB vs Brixelle 400 µM, p=0.0015). Therefore, it was confirmed that, when treated with Brixelle after inducing inflammation, a statistically significant decrease in the expression of TSLP was showed.

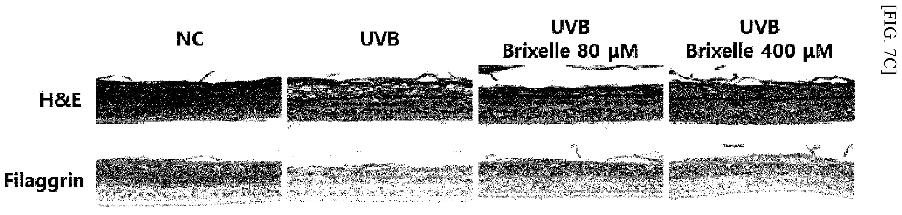

It was intended to confirm the tissue pathology and expression of inflammation and skin barrier function markers in the artificial skin model through H&E staining and filaggrin immunohistochemical staining. Among the skin barrier function markers, filaggrin is an essential factor in regulating epidermal homeostasis and is a component of the lipid envelope within the stratum corneum of the skin, performing moisturizing and barrier functions of the skin. Through H&E staining, it was confirmed that the artificial skin model was formed of four layers (stratum corneum, granular layer, stratum spinosum, and basal layer) like real skin. When inflammation was induced through UV irradiation, the number of stained cell nuclei was reduced compared to the negative control, and it was confirmed that the stratum corneum, which is supposed to be layered, have fallen off. However, in the artificial skin model treated with Brixelle, there was no change in the number of stained cell nuclei compared to the inflammation-induced group, and the stratum corneum showed a normal layered form. Through immunohistochemical staining, the expression of filaggrin was confirmed in the granular layer and the stratum corneum among the four layers of the artificial skin model. In the inflammation-induced group, the expression of filaggrin was decreased by UV irradiation, but in the artificial skin model treated with Brixelle, the decrease in filaggrin expression was confirmed to be suppressed (FIG. 7C).

Summarizing the above results, it was demonstrated that Brixelle could effectively suppress the inflammatory response in the human skin tissue model induced through UV irradiation, recover damaged skin barrier function, and help improve histology.

### 5. Results of validation of Brixelle in atopic dermatitis mouse model (1)

### (1) Experimental method

An HDM-induced atopic dermatitis mouse model was created by applying HDM antigen ointment to the back and ear skin of 6 to 10-week-old NC/Nga female mice a total of 7 times on Days 0, 7, 10, 14, 17, 21 and 24. With respect to a normal group in which HDM antigen was not applied (Normal group), as negative control groups, a group in which no additional substances were applied in the process of inducing atopic dermatitis (No Treatment group) and a group in which excipients were applied to the skin (Vehicle group), and as a positive group, a group in which the previously-approved drug Maxidex was applied (Dexamethasone group, 0.1% Dexamethasone, 62.5 mg/animal), the test substance Brixelle (7.5, 15, and 30 mg/kg) was applied to the skin every day from Day 7, thus to evaluate its effectiveness. Clinical symptoms were evaluated during the process of inducing atopic dermatitis, and skin tissues were secured after the survival test was completed for further analysis.

To quantify the clinical symptoms of atopic dermatitis, for four (4) clinical index items including 1) erythema/hemorrhage, 2) scarring/dryness, 3) edema and 4) excoriation/maceration (erosion), a clinical score was measured on a Likert scale out of 3. H&E staining was performed to observe histological changes in the skin for atopic dermatitis, while dihydroethidium (DHE) staining was performed to measure ROS in skin tissues. Further, in order to identify the mast cells and eosinophils infiltrated in the skin tissues, Toluidine blue staining and Congo red staining were performed, respectively. The results of the graph were expressed as mean ± SEM. The statistical analysis was performed using the GraphPad program to confirm statistical significance with the Vehicle group through one-way ANOVA, followed by Dunnett's multiple comparison method (*p<0.05, **p<0.01, ***p<0.001, ****p< 0.0001).

### (2) Results of experiment

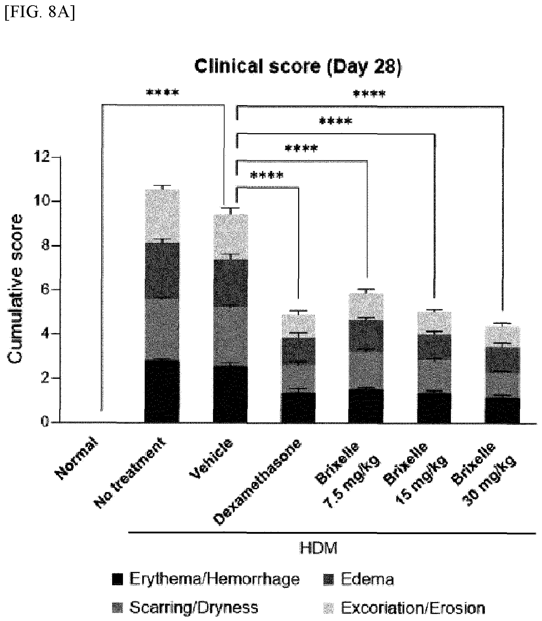

As a result of confirming clinical symptoms at the end of the survival test, crust was formed together with redness and edema due to induction of atopic dermatitis, and secondary skin damage due to itching was clearly observed. As a result of clinical index evaluation, it was confirmed that the Dexamethasone treatment group, which is a previously-approved drug, and the Brixelle treatment group showed a statistically significant improvement effect in clinical symptoms compared to the Vehicle group. Further, the concentration-dependent effect of Brixelle could be confirmed (FIG. 8A. Vehicle vs Dexamethasone, 9.44±0.77 vs 4.90±0.76, p<0.0001; Vehicle vs Brixelle 7.5 mg/kg, 9.44±0.77 vs 5.90±0.47, p<0.0001; Vehicle vs Brixelle 15 mg/kg, 9.44±0.77 vs 5.05±0.32, p<0.0001; Vehicle vs Brixelle 30 mg/kg, 9.44±0.77 vs 4.40±0.48, p<0.0001).

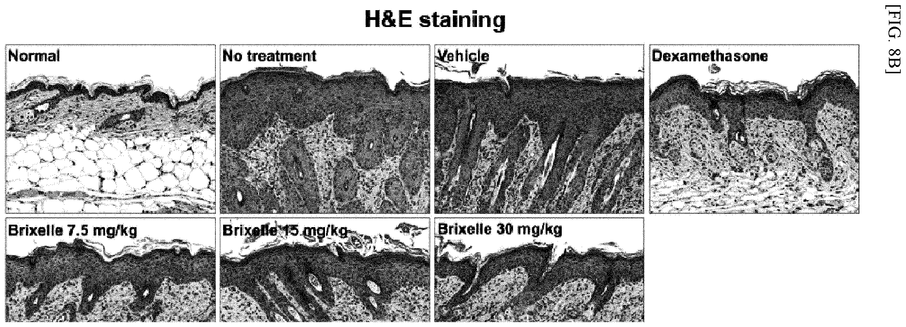

As a result of confirming histological changes in the skin through H&E staining, increased epidermal thickness and hyperkeratinization were observed as the HDM antigen ointment was treated. Further, increased angiogenesis within the dermis, tissue edema, and infiltration of inflammatory cells were confirmed. When compared to the Vehicle group, it could be confirmed that the Dexamethasone treatment group and the Brixelle treatment group showed the decreased epidermal thickness and an effect of reducing hyperkeratinization (FIG. 8B).

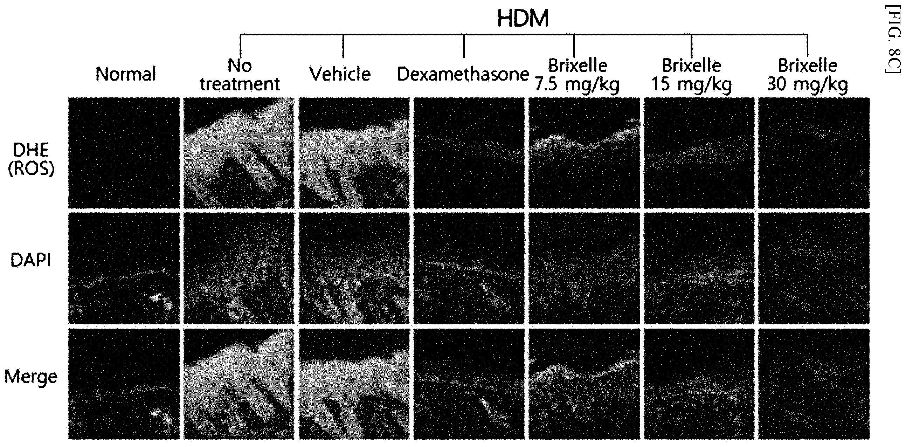

To determine whether the antioxidant activity of Brixelle could have an effect on improving atopic dermatitis symptoms, the expression of ROS in the skin tissues was confirmed. As a result of measuring ROS in the skin tissues through DHE staining, a strong DHE staining intensity was confirmed in the epidermis and dermis in the negative control group compared to the normal group, and a decrease in DHE staining intensity was observed in the Dexamethasone treatment group and Brixelle treatment group (FIG. 8C).

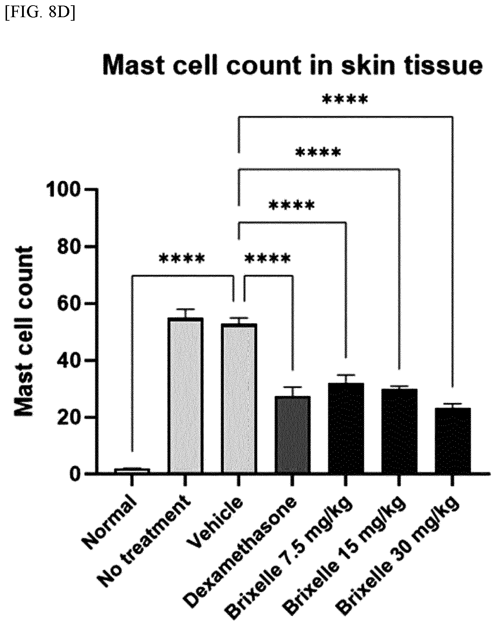

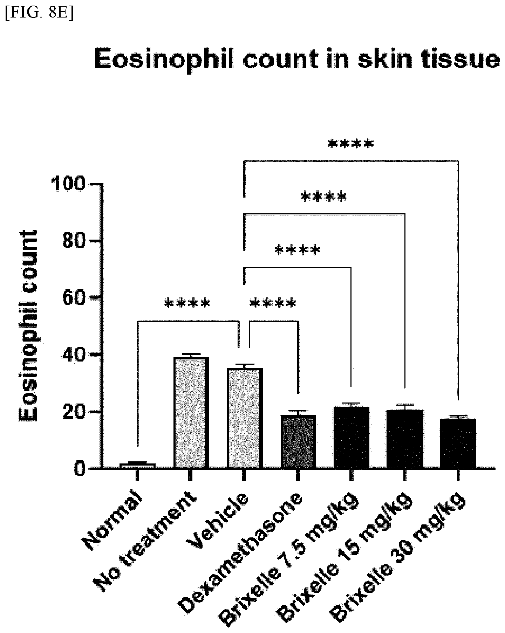

Additional staining was performed to confirm the infiltration of mast cells and eosinophils, which are inflammatory cells known to be associated with the symptoms of atopic dermatitis. The mast cells and eosinophils secrete histamine and cytotoxic substances, respectively, and thus are immune cells that participate in allergic symptoms. As a result of quantifying the mast cells through Toluidine blue staining, it was confirmed that the Dexamethasone treatment group and the Brixelle treatment group showed a statistically significant decrease in mast cell infiltration as compared to the Vehicle group (FIG. 8D, Vehicle vs Dexamethasone, 52.69±2.19 vs 27.38±3.19, p<0.0001; Vehicle vs Brixelle 7.5 mg/kg, 52.69±2.19 vs 32.19±2.72, p<0.0001; Vehicle vs Brixelle 15 mg/kg, 52.69±2.19 vs 29.81±1.11, p<0.0001; Vehicle vs Brixelle 30 mg/kg, 52.69±2.19 vs 23.19±1.57, p<0.0001). Further, when eosinophils were quantified through Congo red staining, it could be confirmed that the Dexamethasone treatment group and the Brixelle treatment group also showed a statistically significant decrease in eosinophil infiltration, as compared to the Vehicle group (FIG. 8E, Vehicle vs Dexamethasone, 35.50±1.37 vs 18.88±1.64, p<0.0001; Vehicle vs Brixelle 7.5 mg/kg, 35.50±1.37 vs 21.56±1.44, p<0.0001; Vehicle vs Brixelle 15 mg/kg, 35.50±1.37 vs 20.88±1.55, p<0.0001; Vehicle vs Brixelle 30 mg/kg, 35.50±1.37 vs 17.56±0.92, p<0.0001).

Based on these results, it could be confirmed that Brixelle, as a powerful antioxidant, prevents clinical symptoms, skin histological changes, and inflammatory responses of atopic dermatitis in a dose-dependent manner.

### 6. Results of validation of Brixelle in atopic dermatitis mouse model (2)

### (1) Experimental method

An HDM-induced atopic dermatitis mouse model was created by applying HDM antigen ointment to the back and ear skin of 6 to 10-week-old NC/Nga female mice a total of 7 times on Days 0, 7, 10, 14, 17, 21 and 24. With respect to a normal group (Normal group) in which HDM antigen was not applied, and as negative control groups, a group in which no additional substances were applied in the process of inducing atopic dermatitis (No Treatment group) and a group in which excipients were applied to the skin (Vehicle group), the test substance Brixelle (30 mg/kg) and the previously-approved drugs Elidel (Elidel group, 1% Pimecrolimus, 62.5 mg/animal), Maxidex (Dexamethasone group, 0.1% Dexamethasone, 62.5 mg/animal) and Eucrisa (Eucrisa group, 2% Crisaborole, 62.5 mg/animal) were applied to the skin every day starting from Day 7, thus to compare its effectiveness. Clinical symptoms were evaluated during the process of inducing atopic dermatitis, and skin tissues were secured after the survival test was completed for further analysis.

To quantify the clinical symptoms of atopic dermatitis, for four (4) clinical index items including 1) erythema/hemorrhage, 2) scarring/dryness, 3) edema and 4) excoriation/maceration (erosion), a clinical score was measured on a Likert scale out of 3. H&E staining was performed to observe histological changes in the skin for atopic dermatitis, while dihydroethidium (DHE) staining was performed to measure ROS in skin tissues. Further, in order to identify the mast cells and eosinophils infiltrated in the skin tissues, Toluidine blue staining and Congo red staining were performed, respectively. To measure IL-4 and IL-13, which are inflammatory mediators known to be highly related to atopic dermatitis, ELISA was performed on the skin tissues. The results of the graph were expressed as mean ± SEM. The statistical analysis was performed using the GraphPad program to confirm statistical significance with the Vehicle group through one-way ANOVA, followed by Dunnett's multiple comparison method (*p<0.05, **p<0.01, ***p<0.001, ****p< 0.0001).

### (2) Results of experiment

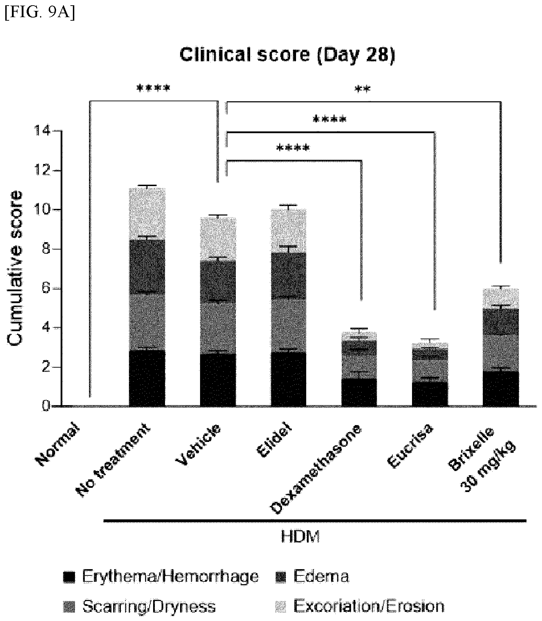

As a result of confirming clinical symptoms at the end of the survival test, crust was formed together with redness and edema due to induction of atopic dermatitis, and secondary skin damage due to itching was clearly observed. As a result of clinical index evaluation, it was confirmed that the treatment groups of Brixelle and two previously-approved drugs (Dexamethasone group and Eucrisa group) showed a statistically significant improvement effect in clinical symptoms as compared to the Vehicle group (FIG. 9A. Vehicle vs Dexamethasone, 9.58±0.64 vs 3.75±0.99, p<0.0001; Vehicle vs Eucrisa, 9.58±0.64 vs 3.25±0.67, p<0.0001; Vehicle vs Brixelle, 9.58±0.64 vs 6.00±0.52, p=0.0034).

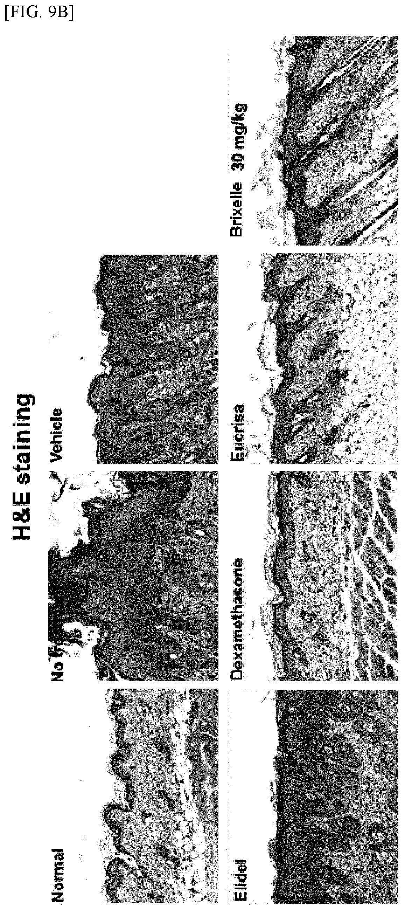

As a result of confirming histological changes in the skin through H&E staining, increased epidermal thickness and hyperkeratinization were observed as the HDM antigen ointment was treated. Further, increased angiogenesis within the dermis, tissue edema, and infiltration of inflammatory cells were confirmed. It could be confirmed that the groups treated with Brixelle and two previously-approved drugs (Dexamethasone group and Eucrisa group) showed the decreased epidermal thickness and an effect of reducing hyperkeratinization as compared to the Vehicle group (FIG. 9B).

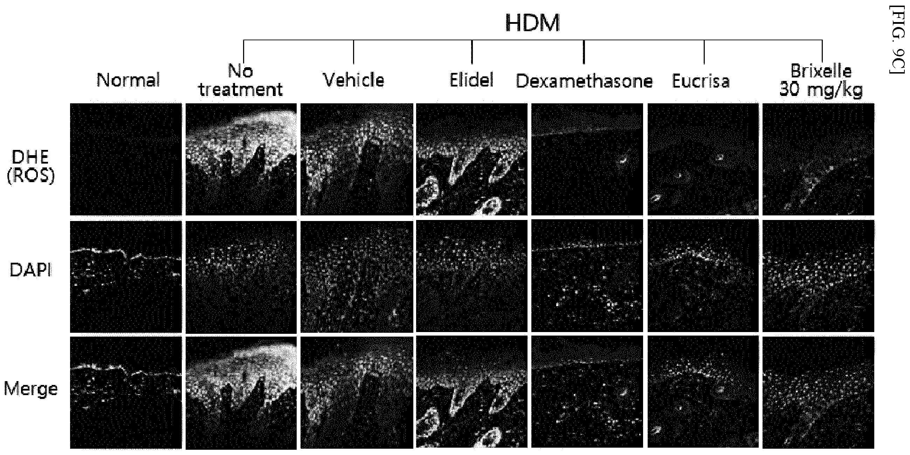

To determine whether the antioxidant activity of Brixelle could have an effect on improving atopic dermatitis symptoms, the expression of ROS in the skin tissues was confirmed. As a result of measuring ROS in the skin tissues through DHE staining, a strong DHE staining intensity was confirmed in the epidermis and dermis in the negative control group compared to the normal group. Further, a decrease in DHE staining intensity was observed in the groups treated with Brixelle and two previously-approved drugs (Dexamethasone group and Eucrisa group) (FIG. 9C).

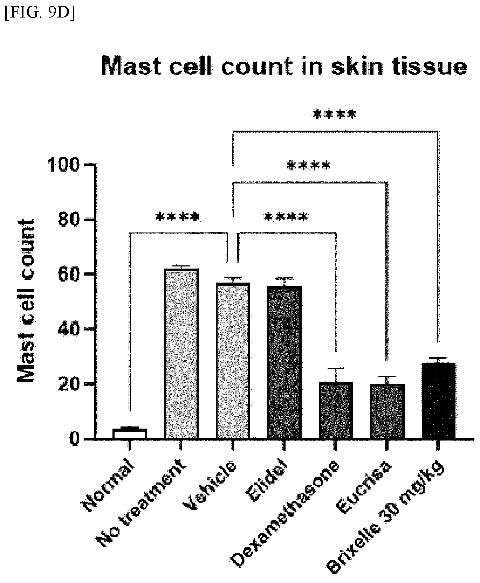

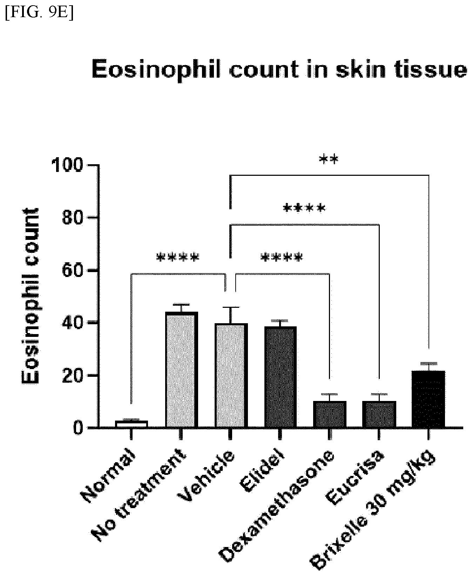

Additional staining was performed to confirm the infiltration of mast cells and eosinophils, which are inflammatory cells known to be associated with the symptoms of atopic dermatitis. The mast cells and eosinophils secrete histamine and cytotoxic substances, respectively, and thus are immune cells that participate in allergic symptoms. As a result of quantifying the mast cells through Toluidine blue staining, it was confirmed that the Brixelle treatment group and the two previously-approved drugs (Dexamethasone group and Eucrisa group) showed a statistically significant decrease in mast cell infiltration as compared to the Vehicle group. (FIG. 9D, Vehicle vs Dexamethasone, 56.81±1.99 vs 20.69±5.25, p<0.0001; Vehicle vs Eucrisa, 56.81±1.99 vs 20.31±2.55, p<0.0001; Vehicle vs Brixelle, 56.81±1.99 vs 27.69±1.94, p<0.0001). Further, when eosinophils were quantified through Congo red staining, it could be confirmed that the Brixelle treatment group and the two previously-approved drugs (Dexamethasone group and Eucrisa group) also showed a statistically significant decrease in eosinophil infiltration as compared to the Vehicle group (FIG. 9E; Vehicle vs Dexamethasone, 40.06±5.95 vs 10.25±2.56, p<0.0001; Vehicle vs Eucrisa, 40.06±5.95 vs 10.31±2.49, p<0.0001; Vehicle vs Brixelle, 40.06±5.95 vs 21.69±2.77, p=0.0027).

Based on these results, it could be seen that Brixelle, as a powerful antioxidant, has a mechanism to prevent clinical symptoms, skin histological changes, and inflammatory responses of atopic dermatitis, similar to the previously-approved drugs Elidel, Dexamethasone and Eucrisa.

### 7. Results of validation of Brixelle in psoriasis mouse model

### (1) Experimental method

An IMQ-induced psoriasis mouse model was created by applying Aldara^{™} (imiquimod, IMQ) cream to the back skin of an 8-week-old C57BL/6 male mouse a total of 7 times from Day 0 to Day 6. With respect to a normal group in which IMQ cream was not applied (Normal group), as negative control groups, a group in which no additional substances were applied in the process of inducing psoriasis (No Treatment group) and a group in which excipients were applied to the skin (Vehicle group), and as a positive control group, a group in which the previously-approved drug Betabate was applied (Clobetasol propionate group, 0.05% Clobetasol propionate, 62.5 mg/animal), the test substance Brixelle (30 mg/kg) was applied to the skin every day from Day 0, thus to evaluate its effectiveness. Clinical symptoms were evaluated during the process of inducing psoriasis, and skin tissues were secured after the survival test was completed for further analysis.

To quantify the clinical symptoms of psoriasis, for three (3) clinical index items including 1) erythema (redness), 2) scaling and 3) skin thickness, a clinical score was measured on a scale out of 4 (modified psoriasis area and severity index, PASI). H&E staining was performed to observe histological changes in the skin due to psoriasis, while DHE staining was performed to measure ROS in the skin tissues. RT-PCR and ELISA were performed on the skin tissues to measure IL-17 and IL-23, which are inflammatory mediators known to be highly associated with psoriasis. The results of the graph were expressed as mean ± SEM. The statistical analysis was performed using the GraphPad program to confirm statistical significance with the Vehicle group through one-way ANOVA, followed by Dunnett's multiple comparison method (*p<0.05, **p<0.01, ***p<0.001, ****p< 0.0001).

### (2) Results of experiment

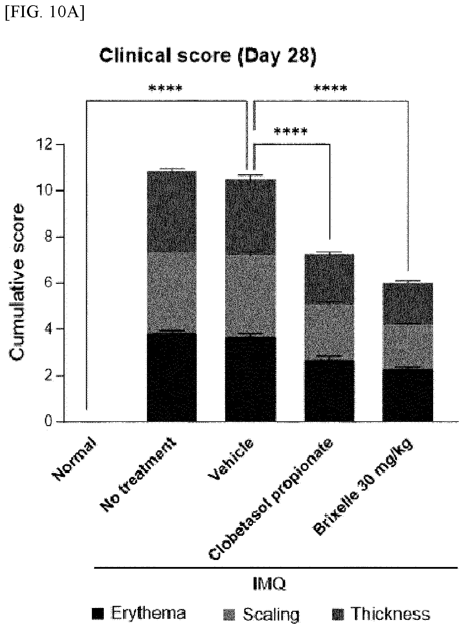

As a result of confirming clinical symptoms at the end of the survival test, formation of crust together with redness and edema were clearly observed due to the induction of psoriasis. As a result of clinical index evaluation, it was confirmed that the Clobetasol propionate treatment group and the Brixelle treatment group showed a statistically significant improvement effect in clinical symptoms as compared to the Vehicle group (FIG. 10A. Vehicle vs Clobetasol propionate, 10.50±0.47 vs 7.25±0.21, p<0.0001; Vehicle vs Brixelle 30 mg/kg, 10.50±0.47 vs 6.00±0.22, p<0.0001).

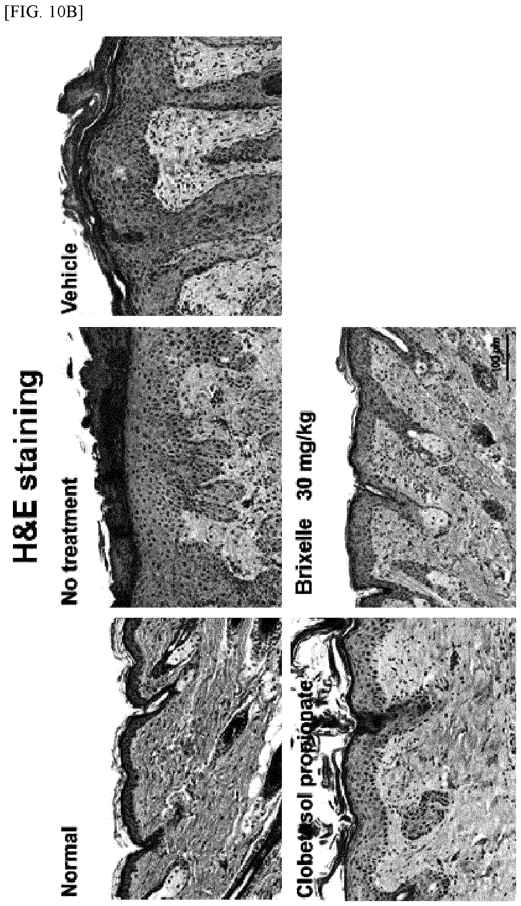

As a result of confirming histological changes in the skin through H&E staining, increased epidermal thickness and hyperkeratinization were observed according to IMQ treatment. The Brixelle treatment group showed a reduction in epidermal thickness and reduced hyperkeratinization compared to the Vehicle group (FIG. 10B).

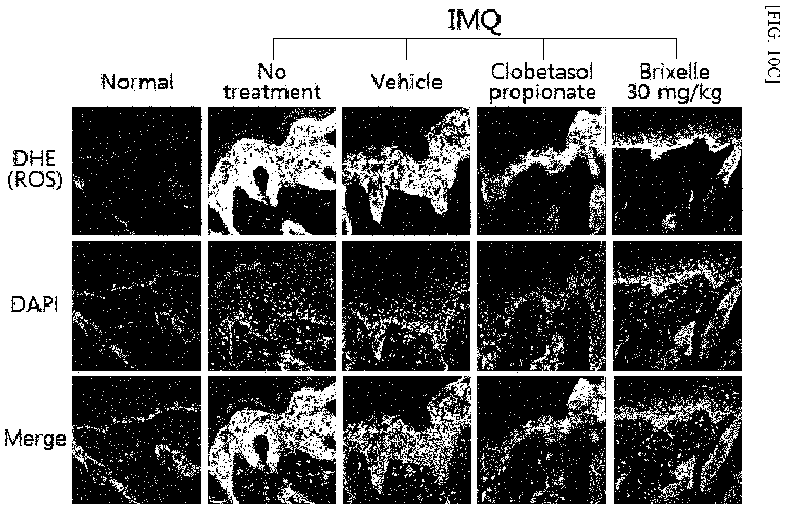

To determine whether the antioxidant activity of Brixelle could have an effect on improving psoriasis symptoms, the expression of ROS in the skin tissues was checked. As a result of measuring ROS in the skin tissues through DHE staining, strong DHE staining intensity was confirmed in the epidermis and dermis in the negative control group compared to the normal group, and a decrease in DHE staining intensity was observed in Brixelle and the positive control group (FIG. 10C).

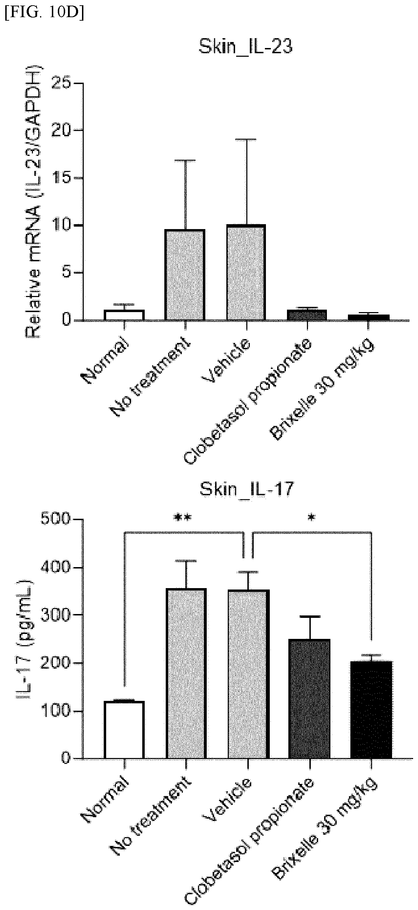

IL-23 and IL-17 as immune mediators related to Th1 and Th17 immune responses, which are important mechanisms in the development of psoriasis symptoms, have a role in initiating and maintaining Th1 and Th17 immune responses and amplifying inflammation, therefore, are being used as a target of psoriasis therapeutics. Macrophages infiltrated in the skin tissues of psoriasis patients express IL-23, allowing γδT cells to express IL-17. Therefore, an analysis to compare the expression of IL-23 and IL-17 in the skin tissues was performed. In the case of IL-23 mRNA expression, the Brixelle treatment group showed a similar reduction effect as the previously-approved drug Clobetasol propionate, although it was not statistically significant as compared to the Vehicle group (FIG. 10D). In the case of IL-17 protein expression, Brixelle showed a statistically significant reduction effect as compared to the Vehicle group (FIG. 10D. Vehicle vs Brixelle 30 mg/kg, 354±36.5 vs 203±15.2, P=0.0378).

Based on these results, it could be seen that Brixelle, as a powerful antioxidant, has a mechanism to prevent clinical symptoms, skin histological changes, and inflammatory responses of psoriasis, which are similar to the previously-approved drug Clobetasol propionate.

### 8. Results of validation of Brixelle in contact dermatitis mouse model

### (1) Experimental method

A DNFB-induced contact dermatitis mouse model was created using 6 to 10-week-old C57BL/6 NC/Nga male mice and Mouse 2, 4 Dinitrofluorobenzene (DNFB). On Days 0 and 1, 0.5% DNFB solution (acetone:olive oil = 4:1) was applied to the back skin to sensitize them, and then on Day 7, 0.3% DNFB solution was applied to the ear skin to induce contact dermatitis. With respect to a normal group in which the DNFB solution was not applied (Normal group), as negative control groups, a group in which no additional substances were applied in the process of inducing contact dermatitis (No Treatment group) and a group in which excipients were applied to the skin (Vehicle group), and as a positive control group, a group in which the previously-approved drug Maxidex was applied (Dexamethasone group, 0.1% Dexamethasone, 62.5 mg/animal), the test substance Brixelle (30 mg/kg) was applied to the skin every day from Day 0, thus to evaluate its effectiveness. Clinical symptoms were evaluated during the process of inducing contact dermatitis, and skin tissues were secured after the survival test was completed for further analysis.

Ear thickness was measured to evaluate edema, a clinical symptom of contact dermatitis. H&E staining was performed to observe histological changes in the skin due to contact dermatitis, while DHE staining was performed to measure ROS in the skin tissues. RT-PCR was performed on the skin tissues to measure TNF-α and IFN-γ, which are inflammatory mediators known to be highly associated with contact dermatitis. The results of the graph were expressed as mean ± SEM. The statistical analysis was performed using the GraphPad program to confirm statistical significance with the Vehicle group through one-way ANOVA followed by Dunnett's multiple comparison method (*p<0.05, **p<0.01, ***p<0.001, ****p< 0.0001).

### (2) Results of experiment

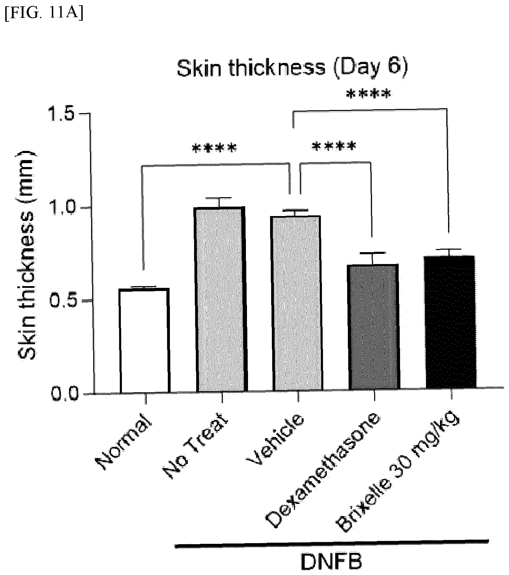

As a result of confirming clinical symptoms at the end of the survival test, edema was clearly observed due to the induction of contact dermatitis. As a result of measuring the thickness of the ear skin, it was confirmed that the Brixelle treatment group showed a statistically significant effect as compared to the Vehicle group (FIG. 11A. Vehicle vs Dexamethasone, 0.94±0.01 vs 0.67±0.02, p<0.0001; Vehicle vs Brixelle 30 mg/kg, 0.94±0.01 vs 0.71±0.01, p<0.0001).

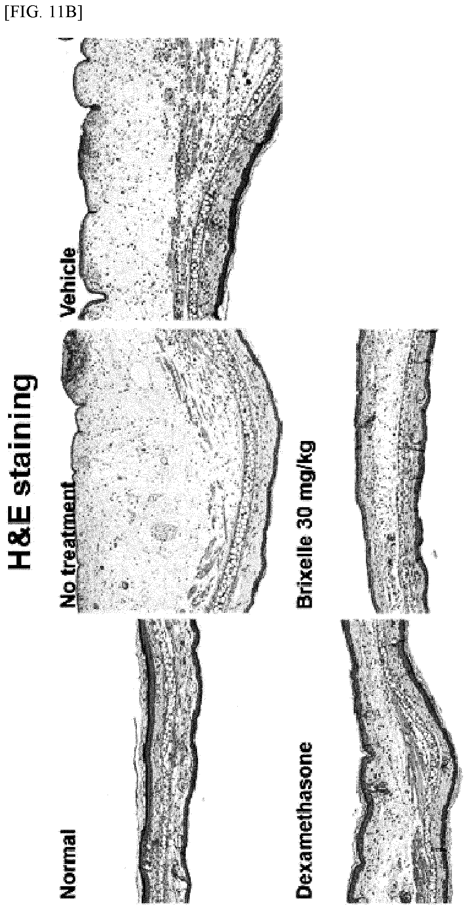

As a result of confirming histological changes in the ear skin through H&E staining, increased skin thickness and tissue swelling were confirmed. The Brixelle treatment group was able to confirm the effect of reduced skin thickness and tissue swelling compared to the Vehicle group (FIG. 11B).

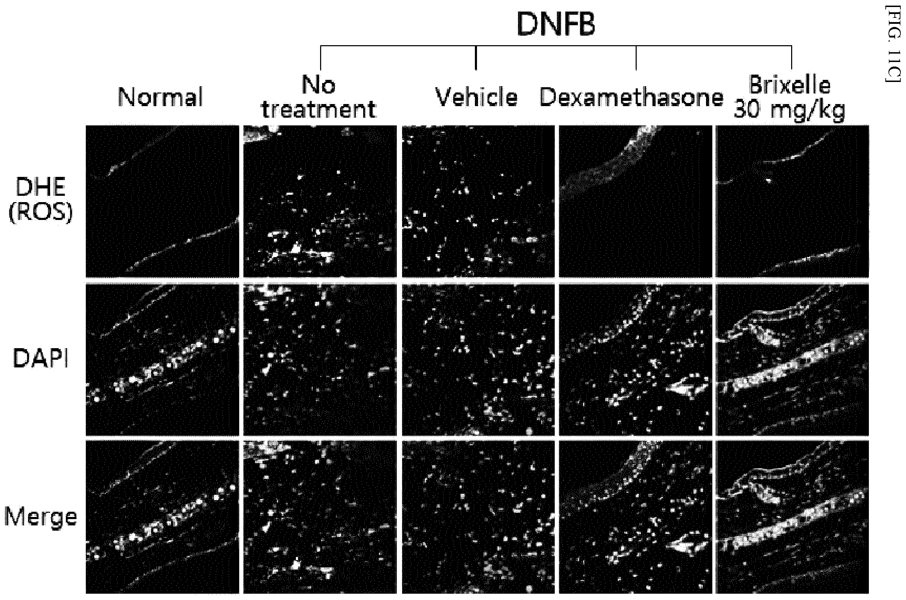

To determine whether the antioxidant activity of Brixelle could have an effect on improving contact dermatitis symptoms, the expression of ROS in ear skin tissues was investigated. As a result of measuring ROS in the skin tissues through DHE staining, strong DHE staining intensity was confirmed in the negative control group compared to the normal group, and a decrease in DHE staining intensity was observed in the Brixelle treatment group and the positive control group (FIG. 11C).

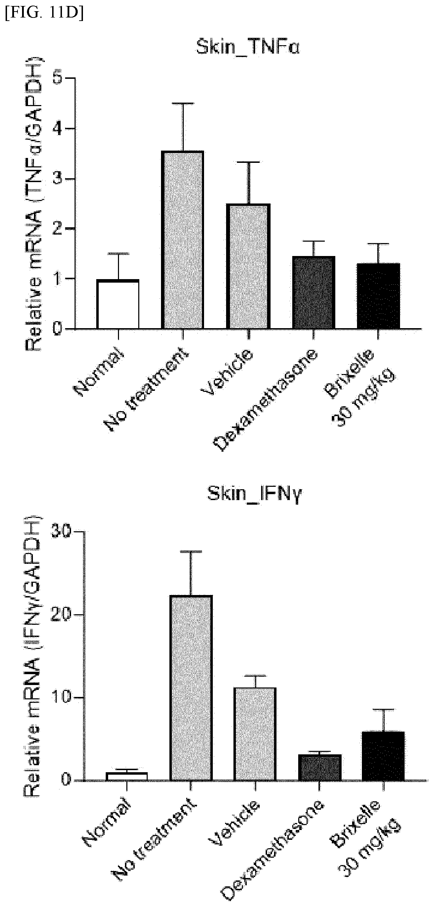

TNF-α and IFN-γ as immune mediators related to the Th1 immune response, which is an important mechanism in the development of symptoms of contact dermatitis, play a role in inducing the Th1 immune response and amplifying inflammation. Regarding the expression of TNF-α and IFN-γ in the skin tissues, RT-PCR was performed to compare mRNA expression. In the case of mRNA expression of the two cytokines, statistical significance could not be confirmed in the Brixelle treatment group as compared to the Vehicle group, but it was confirmed that it showed a similar reduction effect as that of Dexamethasone, the previously-approved drug (FIG. 11D).

Based on these results, it could be seen that Brixelle, as a powerful antioxidant, has a mechanism to prevent clinical symptoms of contact dermatitis, skin histological changes, and inflammatory responses, similar to the previously-approved drug Dexamethasone.

### 9. Results of confirming Brixelle's skin toxicity

### (1) Experimental method

It was intended to determine the effect of Brixelle on the skin tissues using a reconstructed human epidermis model (KeraSkin^{™}) and a reconstructed human cornea-like epithelium model (MCTT HCE^{™}). After treating the reconstructed human epidermis model and reconstructed human cornea-like epithelium model, which are artificial tissues that mimic human skin and cornea, with 3 concentrations of Brixelle (10, 25 and 50 mM), in association with Brixelle, a skin irritation test to confirm a possibility of irritation to the skin; an eye irritation test to confirm a possibility of irritation to the ocular mucous membrane; a skin sensitization test to determine whether there is a possibility of activating the skin's immune system; a skin phototoxicity test to determine whether there is a possibility that UVA changes into a substance irritating the skin; and a skin micronucleus test to determine whether there is a possibility of causing genotoxicity in the skin tissues were performed, respectively.

### (2) Results of experiment

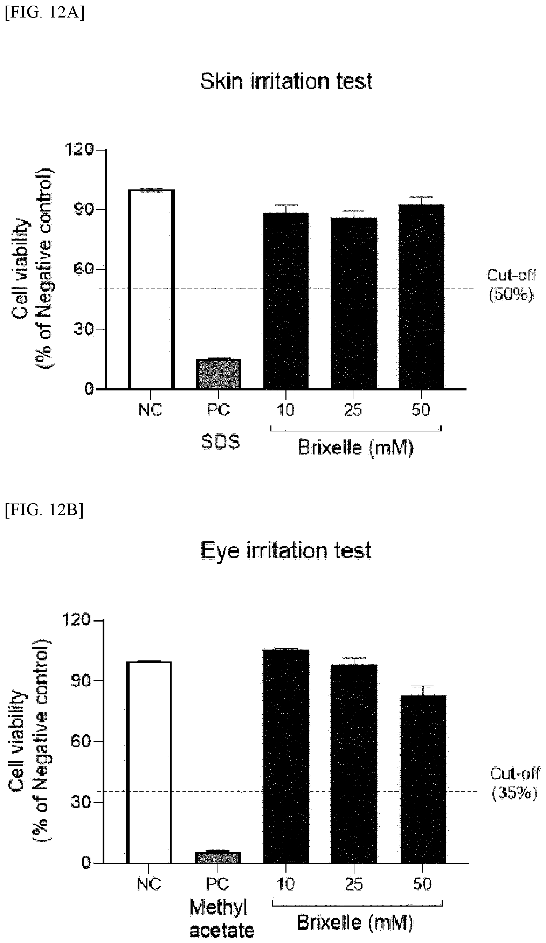

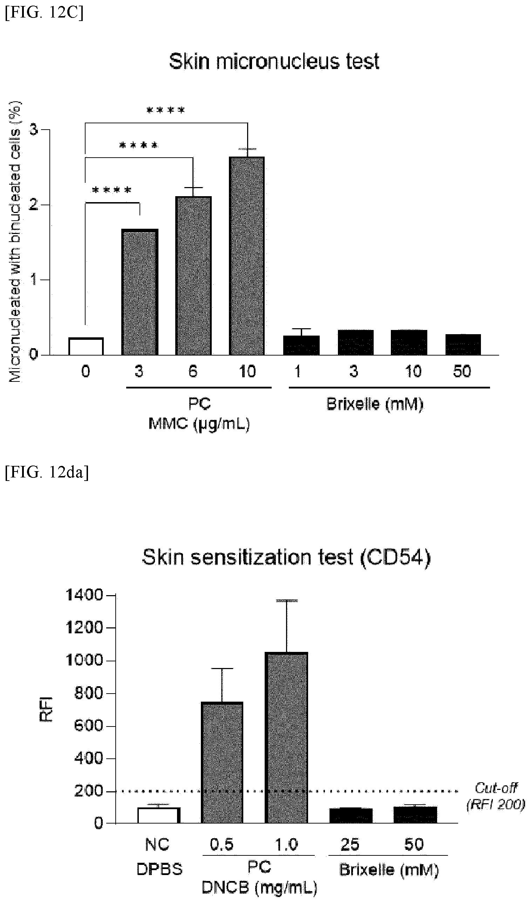

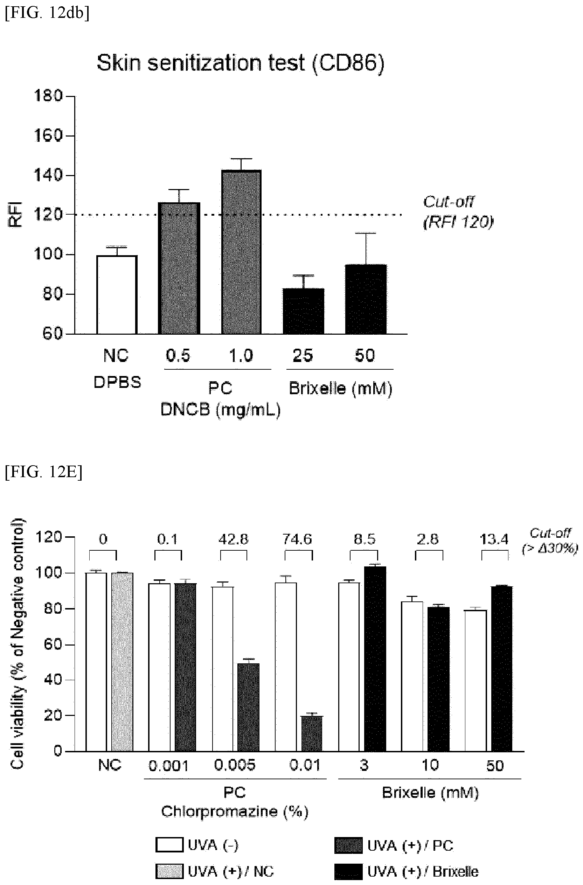

In the skin irritation test to confirm the possibility of the test substance irritating the skin, Brixelle was confirmed to be non-irritating by exhibiting a cell survival rate of 50% or more at up to the concentration of 50 mM (FIG. 12A).

In the eye irritation test to confirm the possibility of the test substance irritating the ocular mucosa, Brixelle was confirmed to be non-irritating by exhibiting a cell survival rate of 35% or more at up to the concentration of 50 mM (FIG. 12B).

In the skin micronucleus test to determine whether the test substance has a possibility to cause genotoxicity in the skin tissues, Brixelle was able to confirm a non-significant micronucleus induction frequency as compared to the negative control at up to the concentration of 50 mM, while it was confirmed to be negative for genotoxicity since the micronucleus induction frequency did not increase in a concentration-dependent manner (FIG. 12C).

In the skin sensitization test to determine whether the test substance has a possibility to activate the skin's immune system, it would be evaluated whether the expression of CD54 and CD86 on the cell surface is increased during differentiation of THP-1 cells which is an immune cell-derived cell line cultured together with the reconstructed human cornea-like epithelium model. Brixelle was demonstrated to express CD54 and CD86 of THP-1 cells cultured together with the same at up to the concentration of 50 mM below 200 RFI and 120 RFI, respectively, compared to the negative control, thereby confirming to be negative for skin sensitization (FIG. 12D (12da and 12db)).

In the skin phototoxicity test to determine whether the test substance has a possibility to change into a substance irritating the skin by UVA, Brixelle showed a difference in cell viability before and after UVA irradiation of less than 30% at up to the concentration of 50 mM, thereby confirming to be negative for skin phototoxicity (FIG. 12E).

Based on these results, it was confirmed that Brixelle, a powerful antioxidant, does not irritate the skin or ocular mucosa, activate the immune system of skin, react with UV to change into a stimulant, or cause genotoxicity.

## Claims

1. A pharmaceutical composition for preventing or treating inflammatory diseases, comprising a compound represented by Formula 1, a solvate thereof, or a pharmaceutically acceptable salt thereof: 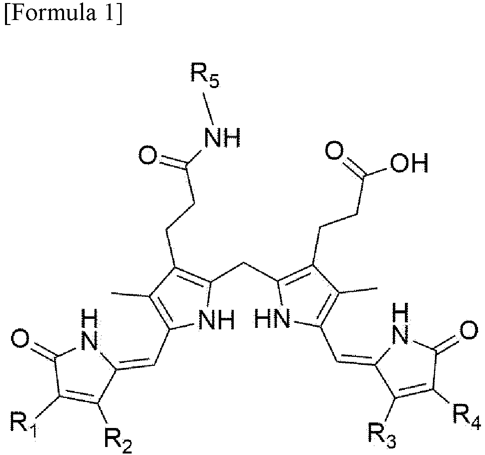
(in Formula 1 above, R₁ and R₄ are vinyl or methyl groups, and R₂ and R₃ are methyl or vinyl groups, but all of them may not be vinyl or methyl groups; and
R₅ is polyethylene glycol (PEG) or derivatives thereof).

2. The pharmaceutical composition for preventing or treating inflammatory diseases according to claim 1, wherein the compound is a compound represented by any one of Formulae 2 to 7:

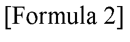

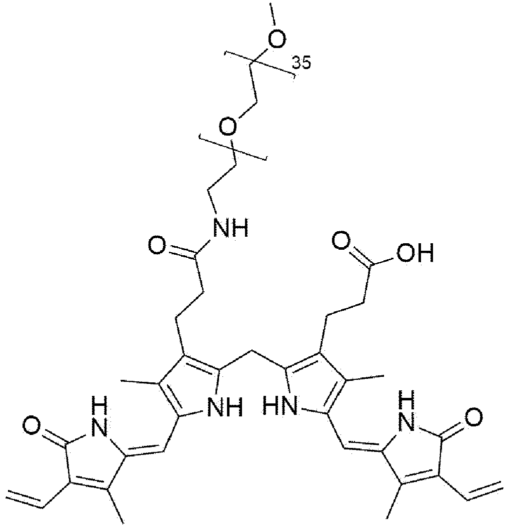

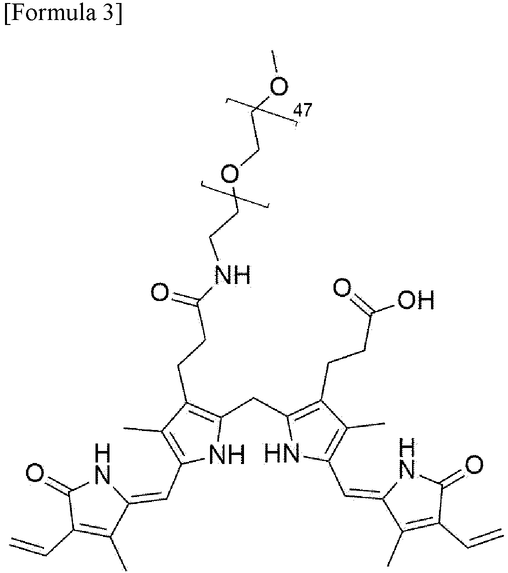

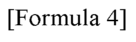

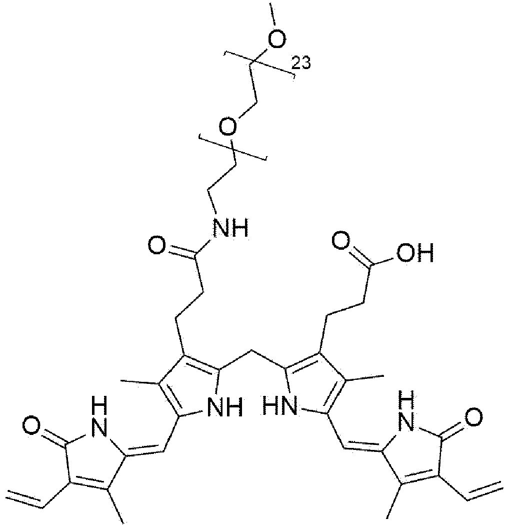

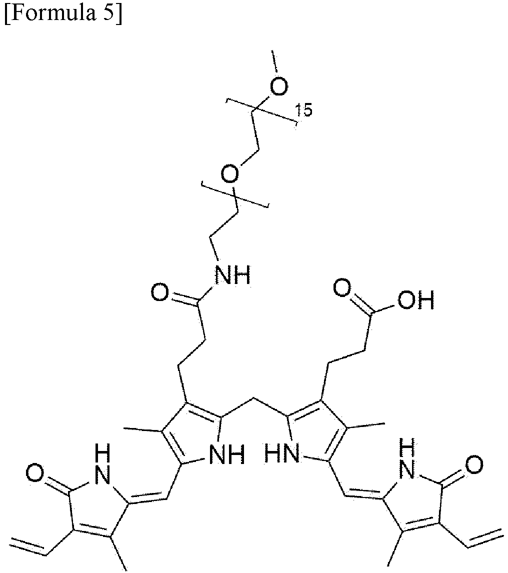

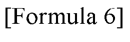

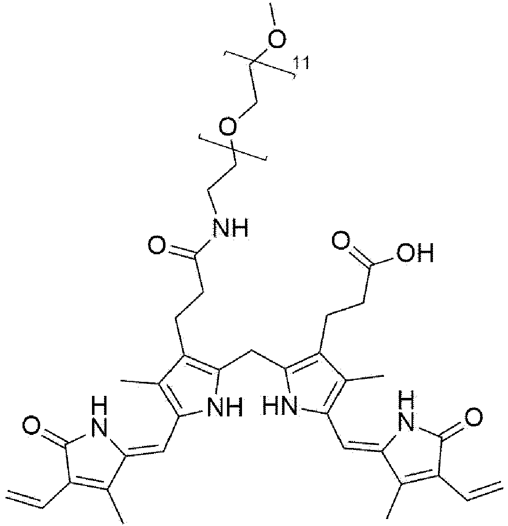

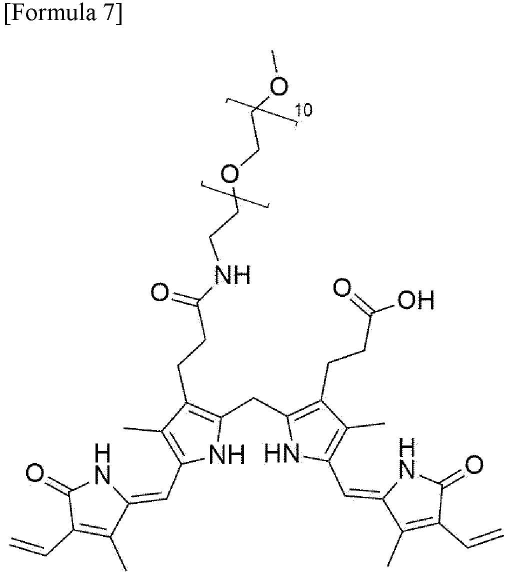

3. The pharmaceutical composition for preventing or treating inflammatory diseases according to claim 1, comprising nanoparticles formed by self-assembly of the compound represented by Formula 1.

4. The pharmaceutical composition for preventing or treating inflammatory diseases according to claim 3, wherein the nanoparticles have a size of 1 to 5000 nm.

5. The pharmaceutical composition for preventing or treating inflammatory diseases according to claim 1, wherein the derivative of polyethylene glycol is selected from the group consisting of methoxy polyethylene glycol (methoxy PEG), succinimide of PEG propionic acid, succinimide of PEG butanoic acid, branched PEG-NHS, PEG succinimidyl succinate, succinimide of carboxymethylated PEG, benzotriazole carbonate of PEG, PEG-glycidyl ether, PEG-oxycarbonylimidazole, PEG nitrophenyl carbonates, PEG-aldehyde, PEG succinimidyl carboxymethyl ester and PEG succinimidyl ester.

6. The pharmaceutical composition for preventing or treating inflammatory diseases according to claim 1, wherein the inflammatory disease is at least one selected from the group consisting of inflammatory skin disease, osteoarthritis, hepatitis, pneumonia, keratitis, gastritis, nephritis, tuberculosis, bronchitis, pleurisy, peritonitis, spondylitis, pancreatitis, inflammatory bowel disease, urethritis, cystitis, inflammatory arteriosclerosis, sepsis, periodontitis, gingivitis and autoinflammatory diseases.

7. The pharmaceutical composition for preventing or treating inflammatory diseases according to claim 6, wherein the inflammatory skin disease is at least one selected from the group consisting of atopic dermatitis, contact dermatitis, psoriasis, seborrheic dermatitis, pruritus, parapsoriasis, urticaria, Lichen planus, sunburn, radiodermatitis, erythema multiforme, erythema nodosum, granuloma annulare, keratosis pilaris, xeroderma, panniculitis, pyoderma gangrenosum, acne, rosacea, lupus erythematosus, pemphigus, diaper dermatitis, pityriasis rosea, alopecia areata, androgenic alopecia, vitiligo and decubitus ulcer.

8. The pharmaceutical composition for preventing or treating inflammatory diseases according to claim 1, wherein the inflammatory disease is caused by increased oxidative stress.

9. A cosmetic composition, comprising a compound represented by Formula 1, a solvate thereof, or a cosmetically acceptable salt thereof: 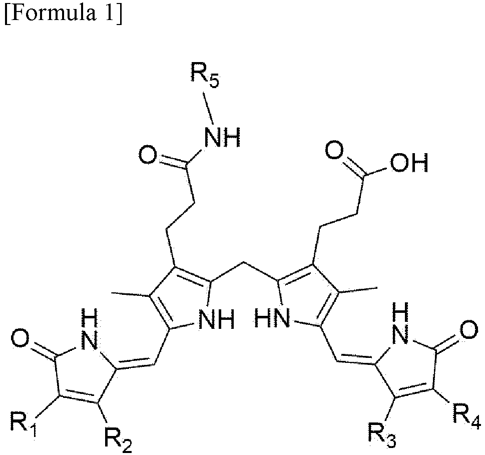
(in Formula 1 above, R₁ and R₄ are vinyl or methyl groups, and R₂ and R₃ are methyl or vinyl groups, but all of them may not be vinyl or methyl groups; and
R₅ is polyethylene glycol (PEG) or derivatives thereof).

10. The cosmetic composition according to claim 9, wherein the compound is a compound represented by any one of Formulae 2 to 7.

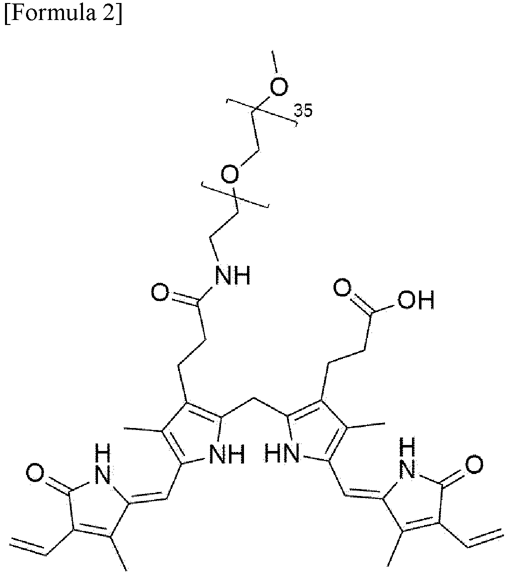

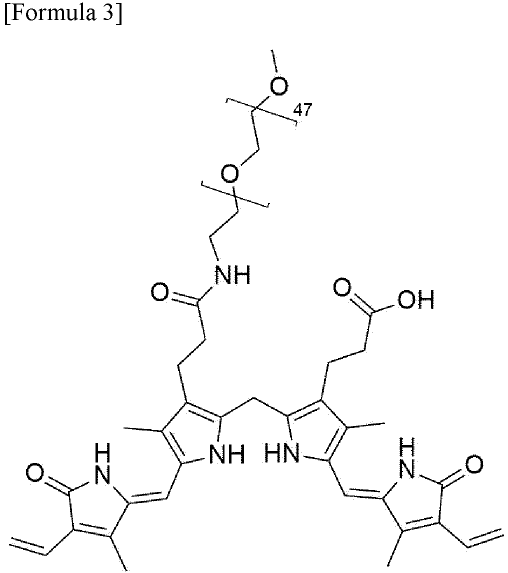

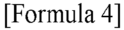

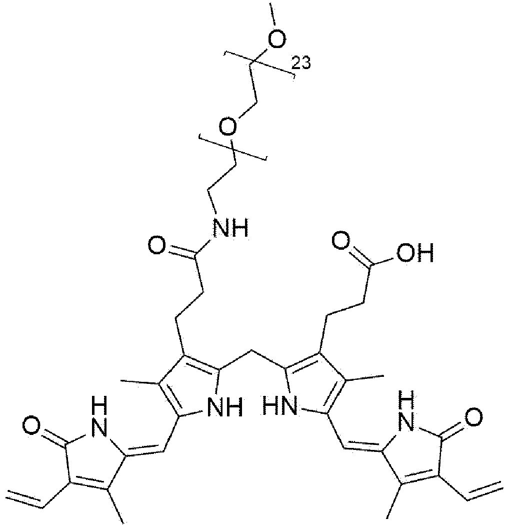

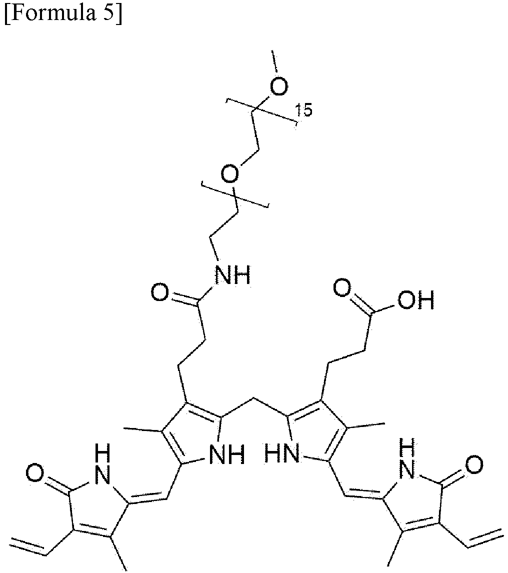

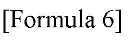

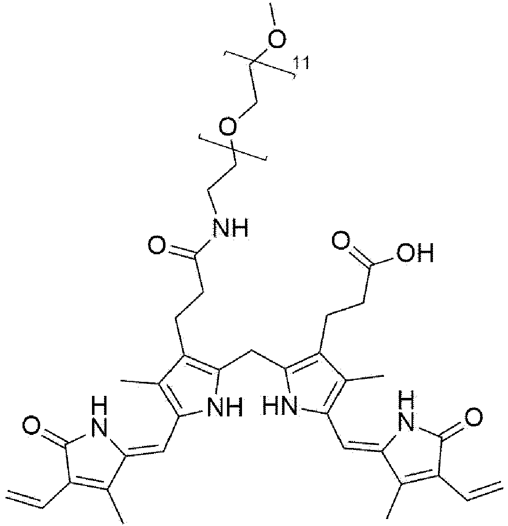

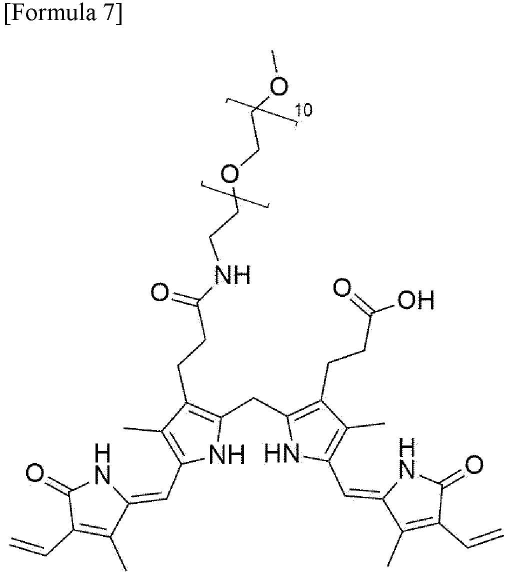

11. The cosmetic composition according to claim 9, comprising nanoparticles formed by self-assembly of the compound represented by Formula 1.

12. The cosmetic composition according to claim 11, wherein the nanoparticles have a size of 1 to 5000 nm.

13. The cosmetic composition according to claim 9, wherein the derivative of polyethylene glycol is selected from the group consisting of methoxy polyethylene glycol (methoxy PEG), succinimide of PEG propionic acid, succinimide of PEG butanoic acid, branched PEG-NHS, PEG succinimidyl succinate, succinimide of carboxymethylated PEG, benzotriazole carbonate of PEG, PEG-glycidyl ether, PEG-oxycarbonylimidazole, PEG nitrophenyl carbonates, PEG-aldehyde, PEG succinimidyl carboxymethyl ester and PEG succinimidyl ester.

14. The cosmetic composition according to claim 9, wherein the cosmetic composition is for anti-inflammatory or anti-oxidation.

15. The cosmetic composition according to claim 9, wherein the cosmetic composition is used to prevent or improve skin inflammation caused by increased oxidative stress.

16. The pharmaceutical composition for preventing or treating inflammatory diseases according to claim 15, wherein skin inflammation caused by the increased oxidative stress is at least one selected from the group consisting of atopic dermatitis, contact dermatitis, psoriasis, seborrheic dermatitis, pruritus, parapsoriasis, urticaria, Lichen planus, sunburn, radiodermatitis, erythema multiforme, erythema nodosum, granuloma annulare, keratosis pilaris, xeroderma, panniculitis, pyoderma gangrenosum, acne, rosacea, lupus erythematosus, pemphigus, diaper dermatitis, pityriasis rosea, alopecia areata, androgenic alopecia, vitiligo and decubitus ulcer.

17. The cosmetic composition according to claim 9, wherein the cosmetic composition is for moisturizing skin, restoring skin barrier function, or preventing skin aging.
